# EUROPEAN PATENT APPLICATION

(11) **EP 4 831 804 A1**
(43) Date of publication of application: **28.10.2026**
(21) Application number: 25741354.2
(22) Date of filing: 03.01.2025
(51) Int. Cl.: G06F 9/451, G06F 3/0482, A63B 24/00, A63B 71/06

(54) **SPORTS ASSISTANCE METHOD AND APPARATUS, AND ELECTRONIC DEVICE**

(30) Priority: 17.01.2024 CN 202410068651
(71) Applicant: Huawei Technologies Co., Ltd., Shenzhen, Guangdong 518129 (CN)
(72) Inventor: MA, Chunhui, Shenzhen, Guangdong 518129 (CN); CHEN, Xiaohan, Shenzhen, Guangdong 518129 (CN); ZHAO, Jie, Shenzhen, Guangdong 518129 (CN); HUANG, Lei, Shenzhen, Guangdong 518129 (CN); WEI, Peng, Shenzhen, Guangdong 518129 (CN)
(74) Representative: Maiwald GmbH
(86) International application number: PCT/CN2025/070341
(87) International publication number: WO 2025/152791

(57) **Abstract**

This application provides an exercise assistance method and apparatus, and an electronic device. The method is applied to a first electronic device, and includes: displaying a first interface, where the first interface includes information about a first user and information about a first target route, the first user is a user of the first electronic device, the first interface further includes information about a second user, the second user and the first user are in a first exercise state, and the first exercise state is a state of exercising along the first target route. According to the exercise assistance method and apparatus, and the electronic device in this application, the electronic device may provide the user with a function of exercise assistance in a group running mode. After joining a group running mode for a route, the user may obtain, through an interface, information about another user who has joined the group running mode for the same route, thereby implementing interaction, and enhancing fun of exercise.

## Description

This application claims priority to Chinese Patent Application No. 202410068651.8, filed with the China National Intellectual Property Administration on January 17, 2024 and entitled "EXERCISE ASSISTANCE METHOD AND APPARATUS, AND ELECTRONIC DEVICE", which is incorporated herein by reference in its entirety.

### TECHNICAL FIELD

This application relates to the field of electronic devices, and more specifically, to an exercise assistance method and apparatus, and an electronic device.

### BACKGROUND

With the development of science and technology, an electronic device may provide a user with various exercise assistance functions, for example, running, swimming, and rope skipping. Among current functions for assisting the user in running, the mode lacks diversity, and only a single-user running mode is supported. In a running process of the user, the electronic device that assists the user in exercise can simply display exercise information of the user, for example, a running route, a running speed, and a heart rate, resulting in a lack of fun during running of the user.

Therefore, how to increase interaction of the user during exercise, and enhance fun of exercise, to improve motivation of the user for exercise is an urgent problem to be resolved currently.

### SUMMARY

This application provides an exercise assistance method and apparatus, and an electronic device. This method increases interaction of a user during exercise, and enhances fun of exercise, thereby improving motivation of the user for exercise.

According to a first aspect, an exercise assistance method is provided. The method is applied to a first electronic device, and includes: displaying a first interface, where the first interface includes information about a first user and information about a first target route, the first user is a user of the first electronic device, the first interface further includes information about a second user, the second user and the first user are in a first exercise state, and the first exercise state is a state of exercising along the first target route.

It should be noted that, that the second user and the first user are in the first exercise state may be understood as that the second user and the first user are exercising in a group running mode for the first target route.

For example, the first interface is an interface of the group running mode for the first target route. Information about the user who is exercising in the group running mode for the route may be displayed on the interface. The information about the user includes information such as a real-time location of the user, current kilometers run by the user, elapsed time, a pace, energy consumed, and a heart rate.

Based on the foregoing solution, the electronic device may provide the user with a function of exercise assistance in a group running mode. After joining a group running mode for a route, the user may obtain, through an interface, information about another user who has joined the group running mode for the same route during exercise, thereby implementing interaction, and enhancing fun of exercise.

With reference to the first aspect, in some implementations of the first aspect, before displaying the first interface, the method further includes: determining the first target route.

Based on the foregoing solution, before the user joins the group running mode, a route needs to be determined as a target route. Manners of determining the target route include determining manually and determining automatically.

With reference to the first aspect, in some implementations of the first aspect, determining the first target route includes: displaying a second interface, where the second interface displays a label of at least one to-be-selected route, and the label of the at least one to-be-selected route includes a label of a first route; and receiving an operation performed by the user on the label of the first route, and determining the first route as the first target route.

It should be noted that a target route needs to be determined, and the target route may be determined by the user through manual selection on an interface. After the target route is determined, the user may join a group running mode for the target route.

With reference to the first aspect, in some implementations of the first aspect, an order of the label of the at least one to-be-selected route is determined based on one or more of the following: slope information of a route, material information of a road surface, personal information of the user, and location information of the user.

It should be noted that, in different scenarios, the order of the label of the at least one to-be-selected route may be different. For example, when the user is currently within a range in which the user frequently exercises and familiar with surrounding routes, the label of the at least one to-be-selected route may be arranged based on an exercise habit of the user, so that a route frequently selected by the user is arranged at the very front. When the user is currently in an unfamiliar environment and unfamiliar with surrounding routes, the label of the at least one to-be-selected route may be sorted based on a distance from the user, so that a route closest to the user is arranged at the very front. When a plurality of route labels are arranged based on a road surface material, road surface material information of each route may be displayed near the route label. For example, a route A is a plastic road surface, and a route B is a cement road surface, so that the user may select the target route based on the road surface material. When a plurality of route labels are arranged based on surrounding environments of the routes, surrounding environment information of each route may be displayed near the route label. For example, a route A is a route inside a city park, and a route B offers a lake view around.

With reference to the first aspect, in some implementations of the first aspect, determining the first target route includes: determining the first target route based on first information, where the first target route belongs to one of at least one to-be-selected route, and the first information includes at least one of the following: an exercise habit of the user and a personal capability of the user.

Based on the foregoing solution, a target route needs to be determined, and the electronic device may automatically match an appropriate route for the user as the target route, so that a process of determining the target route is not perceived, and exercise experience of the user in the group running mode is further improved.

With reference to the first aspect, in some implementations of the first aspect, before determining the first target route based on the first information, the method further includes: determining the at least one to-be-selected route based on second information, where the second information includes one or more of the following: slope information of a route, material information of a road surface, a surrounding environment of a route, location information of the user, and route information of the user.

It should be noted that the to-be-selected routes may be a plurality of routes near a current location of the user, and the to-be-selected routes may be routes preset by the system. Manners of determining the at least one to-be-selected route based on the second information include but are not limited to the following several manners:
Manner 1: A route within a specific range away from the user is determined as the to-be-selected route based on location information of the user (for example, a current location of the user).
Manner 2: A route within a specific range from a running route of the user is determined as the to-be-selected route based on route information of the user (for example, a running route of the user within a time threshold).
Manner 3: A route within a specific range from a running route of the user is determined as the to-be-selected route based on route information of the user (for example, a running route of the user when a distance threshold is reached).

With reference to the first aspect, in some implementations of the first aspect, the method further includes: if a distance between the first user and the first target route is greater than or equal to a distance threshold, outputting a first prompt, where the first prompt indicates the user to return to the first target route.

Optionally, the first prompt may be navigation information.

Based on the foregoing solution, when the user joins a group running mode for a route and may possibly deviate from the route during running, the electronic device may guide the user to return to the correct route through voice or display interaction.

With reference to the first aspect, in some implementations of the first aspect, the first prompt includes a return route, and the return route indicates the user to return to the first target route.

For example, the return route includes navigation information such as a route direction and a distance.

With reference to the first aspect, in some implementations of the first aspect, before outputting the first prompt, the method further includes: outputting a second prompt, where the second prompt is used to prompt the user whether to return to the first target route.

Optionally, the second prompt may be prompt information displayed on the electronic device, or may be an alert tone sent by the electronic device.

Optionally, before outputting the first prompt, the method further includes: determining a return route based on location information of the first user.

It should be noted that, when a deviation from the route occurs, a plurality of routes between a current location of the electronic device and the target route may be determined. From the plurality of routes, a route that requires minimal backtracking and enables the user to return to the target route as quickly as possible is selected as the return route.

With reference to the first aspect, in some implementations of the first aspect, the method further includes: if a distance between the first user and the first target route is greater than or equal to a distance threshold, displaying a third interface, where the third interface includes the information about the first user and information about a second target route, and the second target route is different from the first target route.

For example, the third interface may be a group running mode interface of the second target route. Similar to the first interface (a group running mode interface of the first target route), the third interface displays the information about the second target route, and further displays information about users who have joined a group running mode for the second target route.

Based on the foregoing solution, when the user joins a group running mode for a route and may possibly deviate from the route during running, the electronic device may switch the user to a group running mode for a new route (the second target route).

With reference to the first aspect, in some implementations of the first aspect, before displaying the third interface, the method further includes: outputting a third prompt, where the third prompt is used to prompt the user whether to select the second target route.

Optionally, the third prompt may be prompt information displayed on the electronic device, or may be an alert tone sent by the electronic device.

With reference to the first aspect, in some implementations of the first aspect, the first interface further includes an icon of the second user, and the icon of the second user is an identifier of the second user; and the method further includes: receiving an operation performed by the user on the icon of the second user, and setting the second user as a catch-up target.

Optionally, the operation performed by the user on the icon of the second user is received, and a fourth prompt is output, where the fourth prompt is used to prompt whether to set the second user as a catch-up target.

Optionally, the first electronic device receives an operation performed by the user to set the second user as a catch-up target, and prompts the user with a time required to catch up with the second user.

Optionally, the fourth prompt may be prompt information displayed on the electronic device, or may be an alert tone sent by the electronic device.

Based on the foregoing solution, information about other users who have joined the group running mode for the same route may be obtained via the electronic device. For example, one of the users is selected as a catch-up target, and the electronic device may display a time required for catching up with the user, thereby increasing fun of the group running mode.

With reference to the first aspect, in some implementations of the first aspect, the first interface further includes an icon of the second user, and the icon of the second user is an identifier of the second user; and the method further includes: receiving an operation performed by the user on the icon of the second user, and setting the second user as a pacer.

Optionally, the operation performed by the user on the icon of the second user is received, and a fifth prompt is output, where the fifth prompt is used to prompt whether to set the second user as a pacer.

Optionally, in response to an operation performed by the user to set the second user as a pacer, the first electronic device may prompt comparison information between the first user and the second user, for example, a comparison between paces of the first user and the second user.

Optionally, the fifth prompt may be prompt information displayed on the electronic device, or may be an alert tone sent by the electronic device.

Based on the foregoing solution, information about other users who have joined the group running mode for the same route is obtained via the electronic device. For example, one of the users is selected as a pacer, and the electronic device may display comparison information between the local user and the pacer, thereby increasing fun of the group running mode.

With reference to the first aspect, in some implementations of the first aspect, the method further includes: receiving third information from a second electronic device, and outputting a sixth prompt, where the sixth prompt is used to prompt the second user to set the first user as a pacer, and the second user is a user of the second electronic device.

For example, the third information may be request information, and is used to request to set the first user as a pacer.

Optionally, the sixth prompt may be prompt information displayed on the electronic device, or may be an alert tone sent by the electronic device.

Based on the foregoing solution, the local user may not only set another user as a pacer, but also be set by another user as a pacer, thereby increasing the fun of the group running mode.

With reference to the first aspect, in some implementations of the first aspect, the method further includes: The second user and the first user are in a race mode; and the first interface further includes multiple or more of the following: comparison information between the first user and the second user; ranking information of the first user; and ranking information of the second user.

For example, the comparison information may include comparison information of an exercise parameter. For example, the comparison information may be a quantity of kilometers that have been run, elapsed running time, and a pace.

Based on the foregoing solution, the group running mode may be further divided into a normal mode and a race mode. In the race mode, related information about the race may be displayed. That is, in different modes, a user requirement is more personally catered to, thereby providing the user with a vivid interaction experience.

With reference to the first aspect, in some implementations of the first aspect, the method further includes: when a positioning signal of the first user is less than a signal threshold, determining location information of the first user based on location information of a plurality of third users, where the plurality of third users are in the first exercise state, exercise information of the plurality of third users remains unchanged within a time threshold, and the time threshold is a period of time before the positioning signal of the first user is less than the signal threshold.

For example, in a period of time before a GPS signal of the first electronic device is weak, information about users with a strong GPS signal (for example, a step frequency and an IMU relative velocity) around the first electronic device is recorded. From the users, a plurality of third users whose step frequency and IMU relative velocity remain constant may be selected as reference users. The location information of the first user is calculated based on the location information of the plurality of third users, where the third users are also users in the group running mode for the target route.

Based on the foregoing solution, in the group running mode, a user with a weak GPS signal cannot obtain accurate location information. In this case, location information of other surrounding users with a strong GPS signal may be used to assist the user with a weak signal in positioning.

According to a second aspect, an electronic device is provided, including one or more processors and one or more memories. The one or more memories store one or more computer programs, and the one or more computer programs include instructions. The electronic device is a first electronic device. When the instructions are executed by the one or more processors, the first electronic device is enabled to perform the following steps: displaying a first interface, where the first interface includes information about a first user and information about a first target route, the first user is a user of the first electronic device, the first interface further includes information about a second user, the second user and the first user are in a first exercise state, and the first exercise state is a state of exercising along the first target route.

With reference to the second aspect, in some implementations of the second aspect, before displaying the first interface, the first electronic device performs the following step: determining the first target route.

With reference to the second aspect, in some implementations of the second aspect, when the instructions are executed by the one or more processors, the first electronic device is enabled to perform the following steps: displaying a second interface, where the second interface displays a label of at least one to-be-selected route, and the label of the at least one to-be-selected route includes a label of a first route; and receiving an operation performed by the user on the label of the first route, and determining the first route as the first target route.

With reference to the second aspect, in some implementations of the second aspect, an order of the label of the at least one to-be-selected route is determined based on one or more of the following: slope information of a route, material information of a road surface, personal information of the user, and location information of the user.

With reference to the second aspect, in some implementations of the second aspect, when the instructions are executed by the one or more processors, the first electronic device is enabled to perform the following step: determining the first target route based on first information, where the first target route belongs to one of at least one to-be-selected route, and the first information includes at least one of the following: an exercise habit of the user and a personal capability of the user.

With reference to the second aspect, in some implementations of the second aspect, before determining the first target route based on the first information, the first electronic device is enabled to perform the following step: determining the at least one to-be-selected route based on second information, where the second information includes one or more of the following: slope information of a route, material information of a road surface, a surrounding environment of a route, location information of the user, and route information of the user.

With reference to the second aspect, in some implementations of the second aspect, when the instructions are executed by the one or more processors, the first electronic device is enabled to perform the following step: if a distance between the first user and the first target route is greater than or equal to a distance threshold, outputting a first prompt, where the first prompt indicates the user to return to the first target route.

With reference to the second aspect, in some implementations of the second aspect, the first prompt includes a return route, and the return route indicates the user to return to the first target route.

With reference to the second aspect, in some implementations of the second aspect, before outputting the first prompt, the first electronic device is enabled to perform the following step: outputting a second prompt, where the second prompt is used to prompt the user whether to return to the first target route.

With reference to the second aspect, in some implementations of the second aspect, when the instructions are executed by the one or more processors, the first electronic device is enabled to perform the following step: if a distance between the first user and the first target route is greater than or equal to a distance threshold, displaying a third interface, where the third interface includes the information about the first user and information about a second target route, and the second target route is different from the first target route.

With reference to the second aspect, in some implementations of the second aspect, before displaying the third interface, the first electronic device is enabled to perform the following step: outputting a third prompt, where the third prompt is used to prompt the user whether to select the second target route.

With reference to the second aspect, in some implementations of the second aspect, the first interface further includes an icon of the second user, the icon of the second user is an identifier of the second user, and when the instructions are executed by the one or more processors, the first electronic device is enabled to perform the following steps: receiving an operation performed by the user on the icon of the second user, and setting the second user as a catch-up target.

With reference to the second aspect, in some implementations of the second aspect, the first interface further includes an icon of the second user, the icon of the second user is an identifier of the second user, and when the instructions are executed by the one or more processors, the first electronic device is enabled to perform the following steps: receiving an operation performed by the user on the icon of the second user, and setting the second user as a pacer.

With reference to the second aspect, in some implementations of the second aspect, when the instructions are executed by the one or more processors, the first electronic device is enabled to perform the following steps: receiving third information from a second electronic device, and outputting a sixth prompt, where the sixth prompt is used to prompt the second user to set the first user as a pacer, and the second user is a user of the second electronic device.

With reference to the second aspect, in some implementations of the second aspect, when the instructions are executed by the one or more processors, the first electronic device is enabled to perform the following steps: The second user and the first user are in a race mode; and the first interface further includes one or more of the following: comparison information between the first user and the second user; ranking information of the first user; and ranking information of the second user.

With reference to the second aspect, in some implementations of the second aspect, when the instructions are executed by the one or more processors, the first electronic device is enabled to perform the following step: when a positioning signal of the first user is less than a signal threshold, determining location information of the first user based on location information of a plurality of third users, where the plurality of third users are in the first exercise state, exercise information of the plurality of third users remains unchanged within a time threshold, and the time threshold is a period of time before the positioning signal of the first user is less than the signal threshold.

According to a third aspect, an exercise assistance apparatus is provided. The apparatus includes a display unit, where the display unit is configured to display a first interface, the first interface includes information about a first user and information about a first target route, the first user is a user of the apparatus, the first interface further includes information about a second user, the second user and the first user are in a first exercise state, and the first exercise state is a state of exercising along the first target route.

With reference to the third aspect, in some implementations of the third aspect, the apparatus further includes a processing unit, and the processing unit is configured to determine the first target route.

With reference to the third aspect, in some implementations of the third aspect, the display unit is specifically configured to display a second interface, where the second interface displays a label of at least one to-be-selected route, and the label of the at least one to-be-selected route includes a label of a first route; and the processing unit is further configured to: receive an operation performed by the user on the label of the first route, and determine the first route as the first target route.

With reference to the third aspect, in some implementations of the third aspect, an order of the label of the at least one to-be-selected route is determined based on one or more of the following: slope information of a route, material information of a road surface, personal information of the user, and location information of the user.

With reference to the third aspect, in some implementations of the third aspect, the processing unit is specifically configured to determine the first target route based on first information, where the first target route belongs to one of at least one to-be-selected route, and the first information includes at least one of the following: an exercise habit of the user and a personal capability of the user.

With reference to the third aspect, in some implementations of the third aspect, the processing unit is further configured to determine the at least one to-be-selected route based on second information, where the second information includes one or more of the following: slope information of a route, material information of a road surface, a surrounding environment of a route, location information of the user, and route information of the user.

With reference to the third aspect, in some implementations of the third aspect, the processing unit is further configured to: if a distance between the first user and the first target route is greater than or equal to a distance threshold, output a first prompt, where the first prompt indicates the user to return to the first target route.

With reference to the third aspect, in some implementations of the third aspect, the first prompt includes a return route, and the return route indicates the user to return to the first target route.

With reference to the third aspect, in some implementations of the third aspect, the processing unit is further configured to output a second prompt, where the second prompt is used to prompt the user whether to return to the first target route.

With reference to the third aspect, in some implementations of the third aspect, the display unit is further configured to: if a distance between the first user and the first target route is greater than or equal to a distance threshold, display a third interface, where the third interface includes the information about the first user and information about a second target route, and the second target route is different from the first target route.

With reference to the third aspect, in some implementations of the third aspect, the processing unit is further configured to output a third prompt, where the third prompt is used to prompt the user whether to select the second target route.

With reference to the third aspect, in some implementations of the third aspect, the first interface further includes an icon of the second user, and the icon of the second user is an identifier of the second user; and the processing unit is further configured to: receive an operation performed by the user on the icon of the second user, and set the second user as a catch-up target.

With reference to the third aspect, in some implementations of the third aspect, the first interface further includes an icon of the second user, and the icon of the second user is an identifier of the second user; and the processing unit is further configured to: receive an operation performed by the user on the icon of the second user, and set the second user as a pacer.

With reference to the third aspect, in some implementations of the third aspect, the processing unit is further configured to receive third information from a second electronic device, and output a sixth prompt, where the sixth prompt is used to prompt the second user to set the first user as a pacer, and the second user is a user of the second electronic device.

With reference to the third aspect, in some implementations of the third aspect, the second user and the first user are in a race mode; and the first interface further includes one or more of the following: comparison information between the first user and the second user; ranking information of the first user; and ranking information of the second user.

With reference to the third aspect, in some implementations of the third aspect, the processing unit is further configured to: when a positioning signal of the first user is less than a signal threshold, determine location information of the first user based on location information of a plurality of third users, where the plurality of third users are in the first exercise state, exercise information of the plurality of third users remains unchanged within a time threshold, and the time threshold is a period of time before the positioning signal of the first user is less than the signal threshold.

According to a fourth aspect, an exercise assistance apparatus is provided, including a processor coupled to a memory. The memory is configured to store a computer program, and the processor is configured to run the computer program, so that the exercise assistance apparatus performs the method according to any one of the first aspect and the possible implementations of the first aspect.

With reference to the fourth aspect, in some implementations of the fourth aspect, the apparatus further includes a transceiver, and the transceiver is configured to receive a signal and/or send a signal.

According to a fifth aspect, a computer-readable storage medium is provided. The computer-readable storage medium stores a computer program, and when the computer program is executed by a computer, the computer is enabled to perform the method according to any one of the first aspect and the possible implementations of the first aspect.

According to a sixth aspect, a computer program product including instructions is provided. When the computer program product is run on a computer, the computer is enabled to perform the method according to any one of the first aspect and the possible implementations of the first aspect.

According to a seventh aspect, a chip is provided. The chip includes a processor and a data interface, and the processor reads, through the data interface, instructions stored in a memory, to perform the method according to any one of the first aspect and the possible implementations of the first aspect.

With reference to the seventh aspect, in some implementations of the seventh aspect, the processor is coupled to the memory through an interface.

With reference to the seventh aspect, in some implementations of the seventh aspect, the chip system further includes the memory. The memory stores a computer program or computer instructions.

### BRIEF DESCRIPTION OF DRAWINGS

FIG. 1 is a diagram of a structure of an electronic device;
FIG. 2 is a block diagram of a software structure of an electronic device according to an embodiment of this application;
FIG. 3 is a diagram of a scenario to which an exercise assistance method according to an embodiment of this application is applicable;
FIG. 4A to FIG. 4L show a group of GUIs according to an embodiment of this application;
FIG. 5A to FIG. 5M show a group of GUIs according to an embodiment of this application;
FIG. 6A to FIG. 6J show a group of GUIs according to an embodiment of this application;
FIG. 7 is a schematic flowchart of a method for automatically determining a running route according to an embodiment of this application;
FIG. 8A to FIG. 8E are diagrams of a group of manners for determining a target route according to an embodiment of this application;
FIG. 9 is a diagram of a method for correcting a running route according to an embodiment of this application;
FIG. 10A to FIG. 10F show a group of GUIs according to an embodiment of this application;
FIG. 11A to FIG. 11E show a group of GUIs according to an embodiment of this application;
FIG. 12 is a diagram of an assisted positioning method according to an embodiment of this application;
FIG. 13A and FIG. 13B show a group of GUIs according to an embodiment of this application;
FIG. 14A to FIG. 14D show a group of GUIs according to an embodiment of this application;
FIG. 15 is a schematic flowchart of an exercise assistance method according to an embodiment of this application;
FIG. 16 is a diagram of an exercise assistance apparatus according to an embodiment of this application; and
FIG. 17 is a diagram of an exercise assistance apparatus according to an embodiment of this application.

### DESCRIPTION OF EMBODIMENTS

The following describes technical solutions of this application with reference to accompanying drawings.

Terms used in the following embodiments are merely intended to describe specific embodiments, but are not intended to limit this application. Terms "one", "a", "the", "the foregoing", "this", and "the one" of singular forms used in this specification and the appended claims of this application are also intended to include expressions such as "one or more", unless otherwise specified in the context clearly. It should be further understood that in the following embodiments of this application, "at least one" and "one or more" mean one, two, or more. The term "and/or" is used to describe an association relationship between associated objects and indicates that three relationships may exist. For example, A and/or B may indicate the following cases: Only A exists, both A and B exist, and only B exists, where A and B may be singular or plural. The character "/" generally indicates an "or" relationship between the associated objects.

Reference to "an embodiment", "some embodiments", or the like described in this specification means that one or more embodiments of this application include a specific feature, structure, or characteristic described with reference to embodiments. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "comprise", "have", and their variants all mean "include but are not limited to", unless otherwise specifically emphasized in another manner.

The following describes an electronic device, a user interface used for the electronic device, and embodiments used for using the electronic device. In some embodiments, the electronic device may be a portable electronic device that further includes another function like a personal digital assistant function and/or a music player function, for example, a mobile phone, a tablet computer, or a wearable electronic device (for example, a smartwatch) having a wireless communication function. An example embodiment of the portable electronic device includes but is not limited to a portable electronic device running iOS^{®}, Android^{®}, Microsoft^{®}, or another operating system. The portable electronic device may alternatively be another portable electronic device, for example, a laptop computer (Laptop). It should be further understood that, in some other embodiments, the electronic device may not be the portable electronic device but a desktop computer.

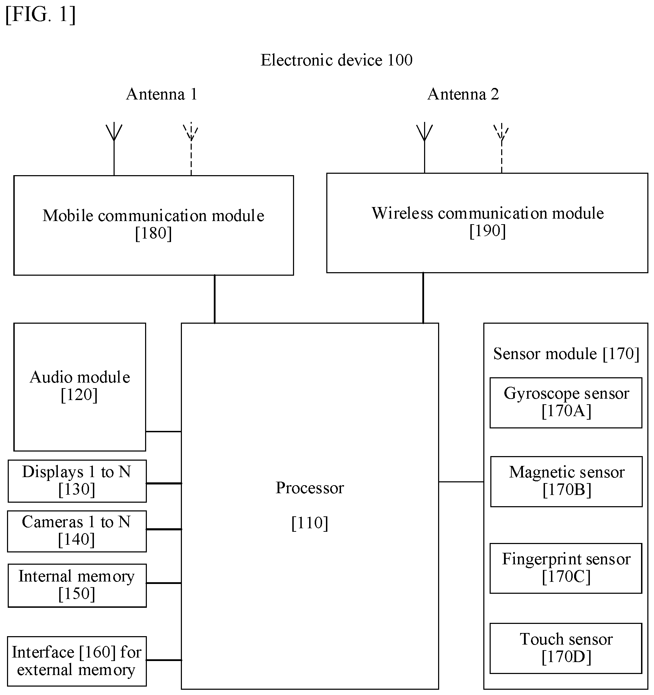

For example, FIG. 1 is a diagram of a structure of an electronic device 100. The electronic device 100 may include a processor 110, an audio module 120, displays 130, a camera 140, an internal memory 150, an interface 160 for external memory, a sensor module 170, an antenna 1, an antenna 2, a mobile communication module 180, a wireless communication module 190, and the like. The sensor module 170 may include a gyroscope sensor 170A, a magnetic sensor 170B, a fingerprint sensor 170C, a touch sensor 170D, and the like. The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), and/or the like.

It may be understood that the structure shown in embodiments of this application does not constitute a specific limitation on the electronic device 100. In some other embodiments of this application, the electronic device 100 may include more or fewer components than those shown in the figure, or some components may be combined, or some components may be split, or different component arrangements may be used. The components shown in the figure may be implemented by hardware, software, or a combination of software and hardware.

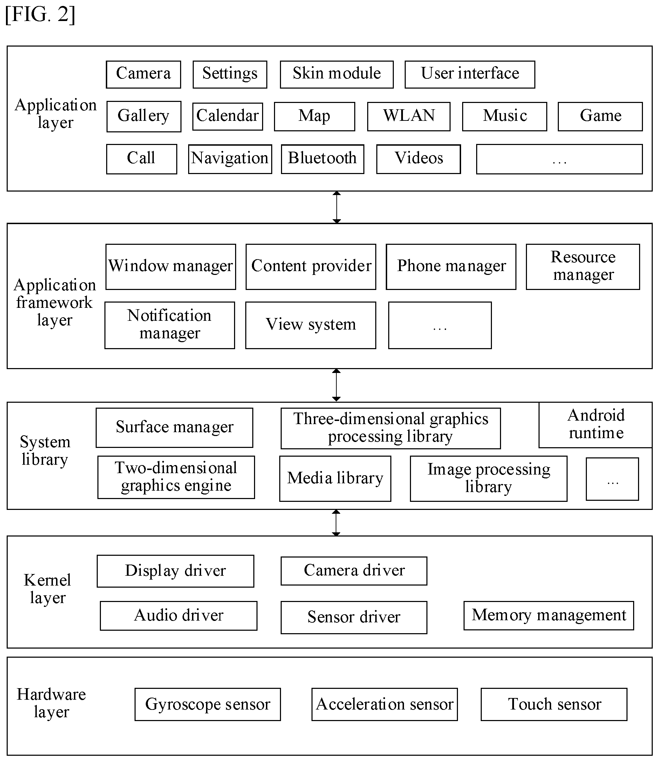

FIG. 2 is a block diagram of a software structure of the electronic device 100 according to an embodiment of this application. For example, an operating system of the electronic device 100 may be an Android (Android) system, a Linux (Linux) system, Microsoft Windows (Windows), iOS (iOS), or HarmonyOS (HarmonyOS). The method provided in this application is particularly applicable to a Linux system or another system derived from a Linux system.

In a layered architecture, software is divided into several layers, and each layer has a clear role and task. The layers communicate with each other through a software interface. In some embodiments, the Android system is divided into four layers: an application layer, an application framework layer, Android runtime (Android runtime) and a system library, and a kernel layer from top to bottom. The application layer may include a series of application packages.

As shown in FIG. 2, the application layer may include Camera, Settings, a skin module, a user interface (user interface, UI), a third-party application, and the like. The third-party application may include Gallery, Calendar, Call, Map, Navigation, WLAN, Bluetooth, Music, Videos, Messages, and the like.

The application framework layer provides an application programming interface (application programming interface, API) and a programming framework for an application at the application layer. The application framework layer may include some predefined functions.

As shown in FIG. 2, the application framework layer may include a window manager, a content provider, a view system, a phone manager, a resource manager, a notification manager, and the like.

The window manager is configured to manage a window program. The window manager may obtain a size of a display, determine whether there is a status bar, perform screen locking, take a screenshot, and the like. The content provider is configured to: store and obtain data, and enable the data to be accessed by an application. The data may include a video, an image, an audio, calls that are made and answered, a browsing history and bookmarks, an address book, and the like.

The view system includes visual controls such as a control for displaying text and a control for displaying an image. The view system may be configured to construct an application. A display interface may include one or more views.

The phone manager is configured to provide a communication function of the electronic device 100, for example, management of a call status (including answering, declining, or the like).

The resource manager provides various resources such as a localized character string, an icon, an image, a layout file, and a video file for an application.

The notification manager enables an application to display, in a status bar, notification information, which may be used for conveying a notification message that may automatically disappear after a short pause without requiring a user interaction. For example, the notification manager is configured to notify download completion, give a message notification, and the like. The notification manager may alternatively be a notification that appears in a top status bar of the system in a form of a graph or scroll bar text, for example, a notification of an application that is run on a background, or may be a notification that appears on a screen in a form of a dialog window. For example, text information is displayed in the status bar, an alert tone is made, the electronic device vibrates, or an indicator blinks.

The Android runtime includes a core library and a virtual machine. The Android runtime is responsible for scheduling and management of the Android system.

The core library includes two parts: a function that needs to be called in Java language and a core library of Android.

The application layer and the application framework layer run on the virtual machine. The virtual machine executes java files of the application layer and the application framework layer as binary files. The virtual machine is configured to implement functions such as object lifecycle management, stack management, thread management, security and exception management, and garbage collection.

The system library may include a plurality of functional modules, for example, a surface manager (surface manager), a media library (media library), a three-dimensional graphics processing library (for example, an OpenGL ES), and a two-dimensional graphics engine (for example, an SGL).

The surface manager is configured to: manage a display subsystem and provide fusion of a 2D layer and a 3D layer for a plurality of applications.

The media library supports playback and recording in a plurality of commonly used audio and video formats, and static image files. The media library may support a plurality of audio and video coding formats, for example, MPEG-4, H.264, MP3, AAC, AMR, JPG, and PNG.

The three-dimensional graphics processing library is configured to implement three-dimensional graphics drawing, image rendering, composition, layer processing, and the like.

The two-dimensional graphics engine is a drawing engine for 2D drawing.

In addition, the system library may further include a status monitoring service module, for example, a physical status recognition module, configured to analyze and recognize a user gesture; and a sensor service module, configured to monitor sensor data uploaded by various sensors at a hardware layer, to determine a physical status of the electronic device 100.

The kernel layer is a layer between hardware and software. The kernel layer includes at least a display driver, a camera driver, an audio driver, a sensor driver, and memory management.

The hardware layer may include various sensors, for example, various sensors described in FIG. 1.

It should be noted that the electronic device described in FIG. 1 and FIG. 2 may be, for example, a device that can have an exercise function, such as a mobile phone terminal, a smartwatch, or smart glasses.

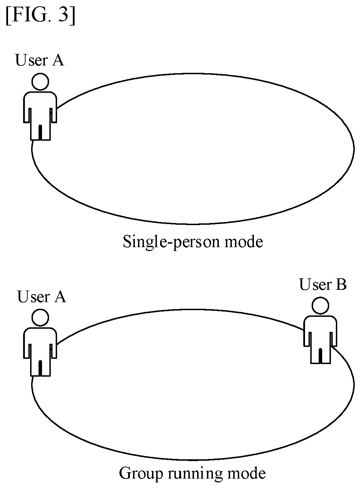

FIG. 3 is a diagram of a scenario to which an exercise assistance method according to this application is applicable. The scenario includes a single-person mode and a group running mode. In a single-person mode scenario, a user A is included. That is, the user A runs alone along a route, and an electronic device may display exercise information of the user A, for example, a running route, a step frequency, an average speed, or a heart rate of the user A. In a group running mode scenario, a plurality of users may be included, for example, a user A and a user B. In this scenario, interaction between the plurality of users may be provided, to increase fun in an exercise process, thereby improving motivation of the users for exercise. In addition, in the group running mode, there may be sub-scenarios such as running route selection, running route correction, and assisted positioning. The following specifically describes the exercise assistance method in the group running mode with reference to the accompanying drawings.

### Scenario 1: Running route selection

It should be noted that the exercise assistance method provided in this application is applicable to a user group running mode. In the group running mode, the user may manually determine a running route, or may automatically determine a running route. The following separately describes the two manners of selecting a running route.

First, manually determine a running route.

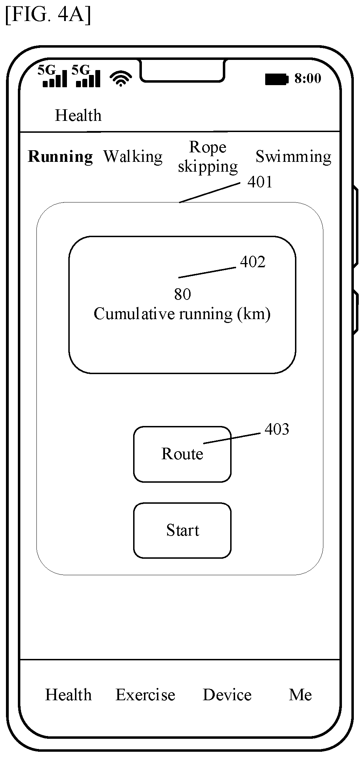

FIG. 4A to FIG. 4L show a group of graphical user interfaces (graphical user interfaces, GUIs) according to an embodiment of this application, to describe a manner of manually determining a running route.

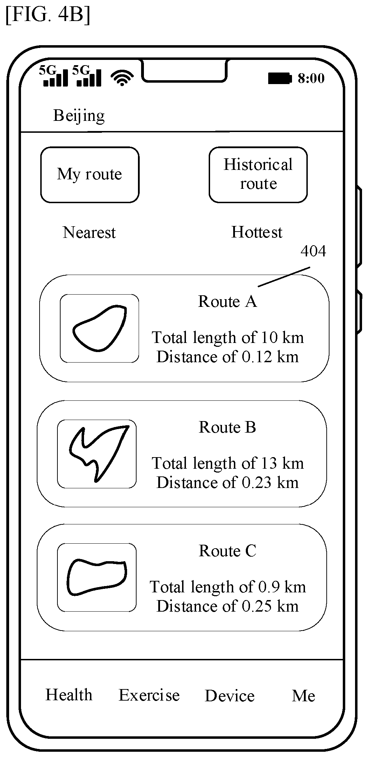

FIG. 4A shows a display interface of a Health application (application, app) of a mobile phone terminal. The bottom of the display interface includes a plurality of controls, for example, a health control, an exercise control, a device control, and a Me control. The current display interface of the Health app corresponds to an interface displayed when the user taps the exercise control. A control is a visual graphical interface element presented to the user, and is a software component included in an application, and controls data processed by the application and an interaction operation about the data. The user may interact with the control through an operation like touch or slide, to read and edit related information of the application. Generally, the control may include visual interface elements such as an icon, a button, a menu, a tab, a text box, and a status bar. In response to an operation (for example, a touch operation or a tap operation) performed by the user on different controls, different interfaces may be displayed on a screen of a terminal device. The top of the display interface further includes a plurality of controls, for example, a running control, a walking control, a rope skipping control, and a swimming control. The display interface corresponds to an interface displayed when the user taps the running control. The display interface further includes prompt information 401, where the prompt information 401 includes cumulative running kilometer information 402, and further includes a route control and a start control. In response to an operation performed by the user on the route control 403, the mobile phone terminal may display a GUI shown in FIG. 4B.

It should be noted that the prompt information used to indicate the user or prompt the user in this embodiment of this application is merely an example for description. The electronic device may indicate the user or prompt the user in another manner. A specific manner of indicating or prompting the user by the electronic device is not limited in embodiments of this application, and may be a manner of displaying prompt information, or may be a manner of playing an alert tone. In the exercise assistance method provided in this embodiment of this application, for example, prompt information is displayed in a form of a pop-up window, or may be displayed in a form of a floating window or the like. A specific display manner of the prompt information is not limited in embodiments of this application.

As shown in FIG. 4B, in response to the operation performed by the user on the route control 403, the mobile phone terminal displays a running route interface. A plurality of route labels are displayed on the interface, and the plurality of route labels may be arranged in a specific order. For example, the plurality of route labels are arranged in order of distance from a current location of the user; or arranged based on a quantity of participants on the route (that is, route popularity); or arranged based on slope information of a route; or arranged based on a road surface material; or arranged based on a surrounding environment of a route; or arranged based on an exercise habit of a local user (a user using the current electronic device). It should be noted that the route labels displayed on the interface respectively correspond to routes recommended by the system for the user, and the user may select any route from the plurality of routes for exercise.

It should be noted that, in different scenarios, an arrangement order of the plurality of route labels may be different. For example, when the user is currently within a range in which the user frequently exercises and familiar with surrounding routes, the plurality of route labels may be arranged based on an exercise habit of the user, so that a route frequently selected by the user is arranged at the very front. When the user is currently in an unfamiliar environment and unfamiliar with surrounding routes, the plurality of route labels may be sorted based on a distance from the user, so that a route closest to the user is arranged at the very front. When a plurality of route labels are arranged based on a road surface material, road surface material information of each route may be displayed near the route label. For example, a route A is a plastic road surface, and a route B is a cement road surface, so that the user may select a target route based on the road surface material. When a plurality of route labels are arranged based on surrounding environments of the routes, surrounding environment information of each route may be displayed near the route label. For example, a route A is a route inside a city park, and a route B offers a lake view around.

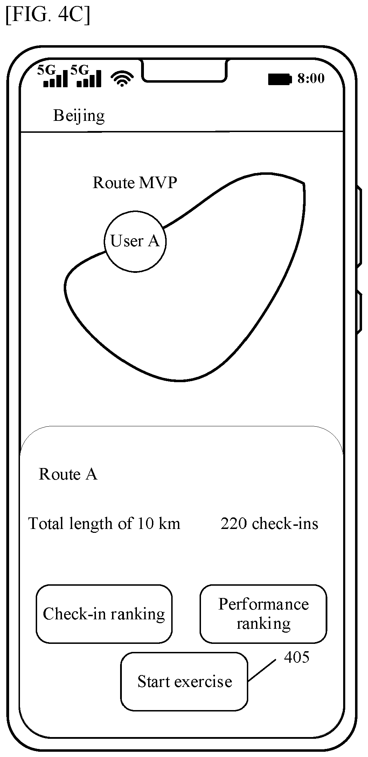

For example, as shown in FIG. 4B, on the running route interface, the plurality of route labels are arranged in order of distance from a current location of the user, and the plurality of route labels include a label of a route A, a label of a route B, and a label of a route C. Each route label displays basic information of the route. For example, the label of the route A indicates that a total length of the route A is 10 km and a distance from the current location of the user is 0.12 km. In response to an operation performed by the user on the label 404 of the route A, the mobile phone terminal may display a GUI shown in FIG. 4C.

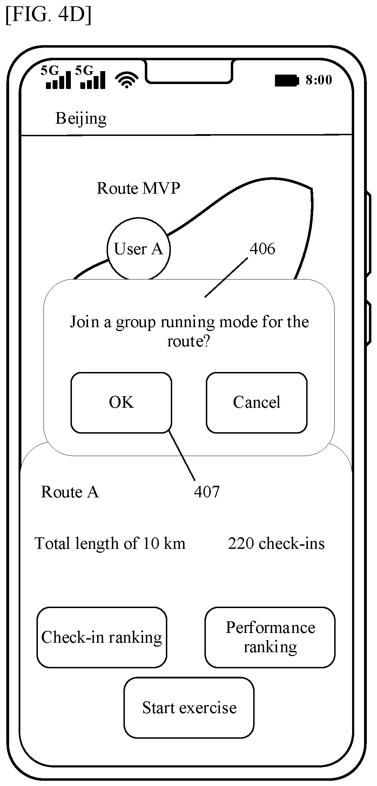

As shown in FIG. 4C, in response to the operation performed by the user on the label 404 of the route A, the mobile phone terminal displays an interface of the route A. The interface displays the route A selected by the user, and may further display basic information of the route A. The basic information of the route A includes a route length, a quantity of check-in users, and the like. For example, the route A has a total length of 10 km and has a total of 220 check-ins. An MVP user of the route is further displayed on the route A. For example, if the user A is displayed as the route MVP of the route A, it may indicate that the user A has the most check-ins on the route A, or the user A has the longest exercise time or the longest exercise distance along the route A, or the user A has the highest step frequency or the fastest pace on the route A. The user may zoom in on the route A to view details of the route A, for example, to view a surrounding environment of the route A, by performing a two-finger swipe operation on the route A. The interface further displays a check-in ranking control, a performance ranking control, and a start exercise control 405. In response to an operation performed by the user on the check-in ranking control, users ranked based on a quantity of check-ins on the route A may be viewed. In response to an operation performed by the user on the performance ranking control, users ranked by at least one of the exercise parameters, such as a step frequency, exercise duration, or an exercise distance, may be viewed. In response to an operation performed by the user on the start control 405, the mobile phone terminal may display a GUI shown in FIG. 4D.

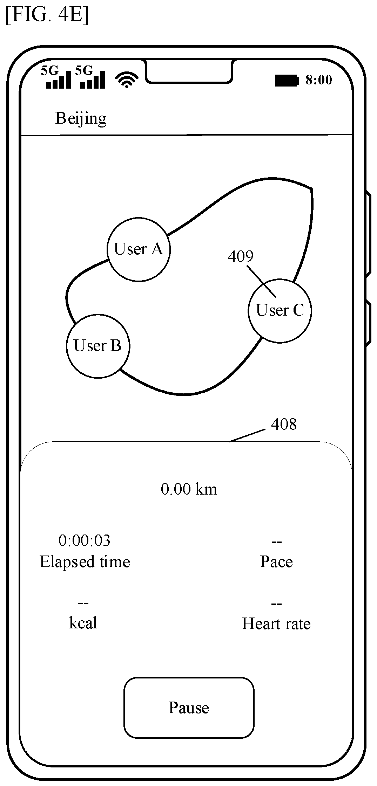

As shown in FIG. 4D, in response to the operation performed by the user on the start control 405, the mobile phone terminal displays an interface of the route A. Optionally, prompt information 406 is displayed on the interface. The prompt information 406 is used to prompt the user whether to join a group running mode for the route. The prompt information 406 includes an OK control and a cancel control. In response to an operation performed by the user on the OK control 407, the mobile phone terminal may display a GUI shown in FIG. 4E.

As shown in FIG. 4E, in response to the operation performed by the user on the OK control 407, the mobile phone terminal displays a group running mode interface. The interface displays basic information of users who have joined the group running mode for the route A. For example, the users who have joined the group running mode for the route A include the user A, a user B, and a user C. The interface displays an icon of the user A, an icon of the user B, and an icon of the user C. It should be noted that the user icon may be displayed on the interface in various manners. In a possible implementation, one or more user icons are displayed on the interface in a form of a list. When the user icons are displayed on the interface in a form of a list, the list may be displayed in an upper left corner, an upper right corner, or another position of the interface. The user icons in the list may be arranged based on parameters such as a pace speed, running duration, and a running distance of the users. Displaying the user icons in a list can provide more parameter information about exercise. Optionally, a display position of the list may be changed based on an operation performed by the user on the list. In another possible implementation, the user icon may alternatively be displayed on the interface based on a specific position of the user. Optionally, a display position of the user icon may be updated with a change of a real-time location of the user. For the user icons in FIG. 4E, near the displayed route A, the icon of the user A, the icon of the user B, and the icon of the user C may each be updated with changes in the respective corresponding users, so that the local user may view in real time real-time exercise positions of the users who have joined the group running mode for the route A, thereby facilitating the local user in setting a catch-up target or a pacer, or implementing another interaction manner. It may be understood that, in some possible implementations, the user interface may display a user icon based on a specific position of the user while displaying the user icons in a form of a list.

Prompt information 408 is further displayed on the group running mode interface, and the prompt information 408 includes current running information of the local user of the mobile phone terminal, for example, current kilometers run, elapsed time, a pace, energy consumed (kilocalorie), and a heart rate.

For example, the current kilometers run may be actual kilometers run by the local user, or may be kilometers run by the local user in the group running mode for the route A. The actual kilometers run by the local user may be greater than the kilometers run by the local user in the group running mode for the route A. For example, the user first runs a distance in a free exercise mode and then runs a distance in the group running mode for the route A, and the actual kilometers run by the local user is a cumulative distance of the distance run in the free exercise mode and the distance run in the group running mode for the route A. For another example, the user first runs a distance in a group running mode for the route B and then runs a distance in the group running mode for the route A, and the actual kilometers run by the local user is a cumulative distance of the distances run in the group running modes of the two routes. In other words, when the current kilometers run are used to accumulate the running distance of the user, an actual running distance of the user may be accumulated when the user exercises freely or switches exercise routes, so that the user may obtain a total running distance at any time, and may adjust an exercise status of the user at any time. When the current kilometers run indicate kilometers run by the user in the group running mode for the route A, the user may obtain running information for the specific exercise scenario, thereby facilitating the user in adjusting a running status in the exercise scenario. If the user successively exercises on a plurality of different routes in a period of time, when the user finishes this exercise session, kilometers run by the user on each of the plurality of routes may be further displayed separately, thereby facilitating the user in adjusting a step frequency, a pace, or the like of the user on each route based on the kilometers run on the plurality of routes.

The elapsed time may indicate actual exercise duration of the local user, or may be exercise duration of the local user in the group running mode for the route A.

The actual exercise duration of the local user may be greater than the exercise duration of the local user in the group running mode for the route A. For example, the user first exercises for a period of time in a free exercise mode and then exercises for a period of time in the group running mode for the route A, and the actual exercise duration of the local user is cumulative duration of exercise duration in the free exercise mode and the exercise duration in the group running mode for the route A. For another example, the user first exercises for a period of time in a group running mode for the route B and then exercises for a period of time in the group running mode for the route A, and the actual exercise duration of the local user is cumulative duration of exercise duration in the group running modes of the two routes. In other words, when the elapsed time represents cumulative exercise duration of the user, even if the exercise route of the user is switched, the exercise duration is accumulated, so that the user can obtain the total exercise duration at any time. When the elapsed time represents the exercise duration of the user in the group running mode for the route A, the user may obtain running information for the specific scenario, thereby facilitating the user in adjusting a running status in the exercise scenario. If the user successively exercises on a plurality of different routes in a period of time, when the user finishes this exercise session, exercise duration of the user on each of the plurality of routes may be further displayed separately, thereby facilitating the user in adjusting a step frequency, a pace, or the like of the user on each route based on the exercise duration on the plurality of routes.

Similarly, the energy consumed may be energy consumed by the user in the group running mode for the route A, or may be energy consumed by the user since the start of the exercise, or may be energy consumed separately by the user during exercise on different routes. For example, if the user consumes a high amount of energy in the group running mode for the route A, the user may choose to start exercising in the group running mode for the route A within limited exercise duration.

Further, the local user may view, through the interface, running information of other users who have joined the group running mode for the route A, where the running information may include real-time location information, a step frequency, a pace, and the like, to implement interactions such as setting a catch-up target or setting a pacer by the local user.

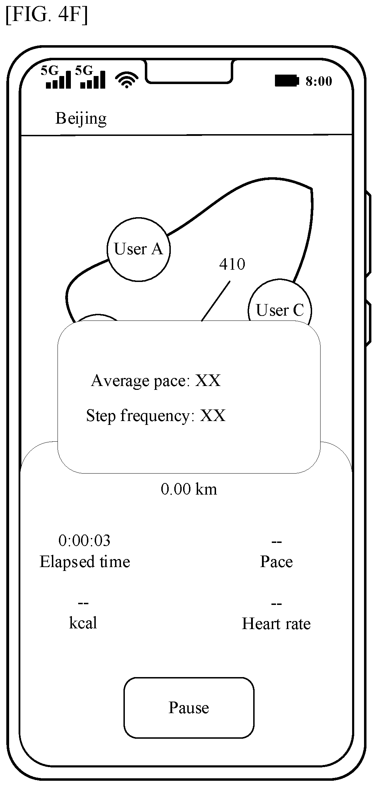

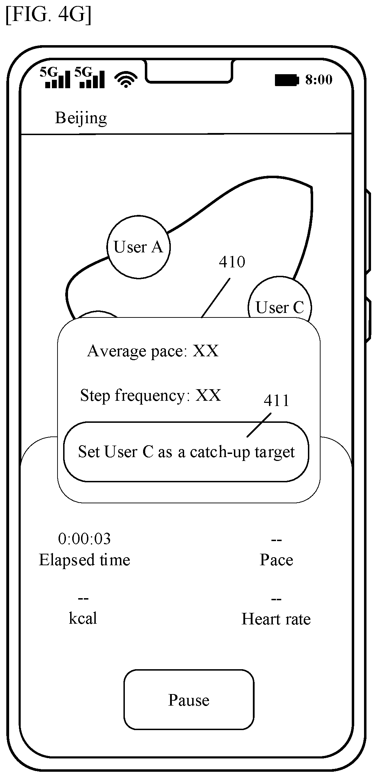

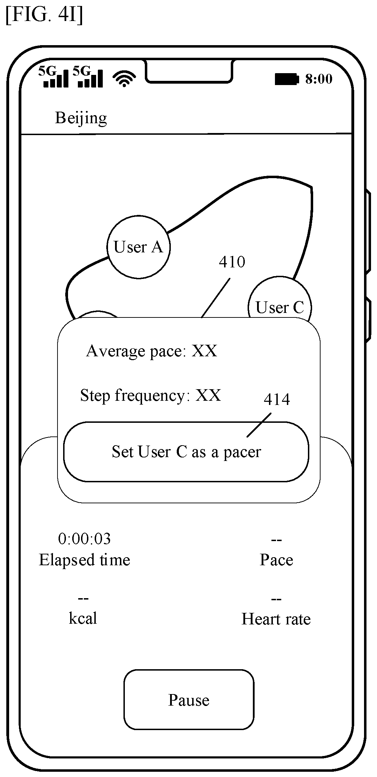

For example, in response to an operation performed by the user on the icon 409 of the user C, the mobile phone terminal may display a GUI shown in FIG. 4F, or display a GUI shown in FIG. 4G, or display a GUI shown in FIG. 4I.

On the group running mode interface, by performing operations on different user icons, the user may obtain running information of a user corresponding to each user icon, and richer interaction scenarios can be achieved. As shown in FIG. 4F, in response to the operation performed by the user on the icon 409 of the user C, the mobile phone terminal displays a group running mode interface. For example, prompt information 410 is displayed on the interface, and the prompt information 410 indicates running information of the user C, for example, an average pace and a step frequency of the user C. Optionally, the prompt information 410 may further indicate relative information between the user C and the local user, for example, a distance between the user C and the local user, or a pace difference between the local user and the user C.

Further, richer interaction scenarios may be further achieved on the interface.

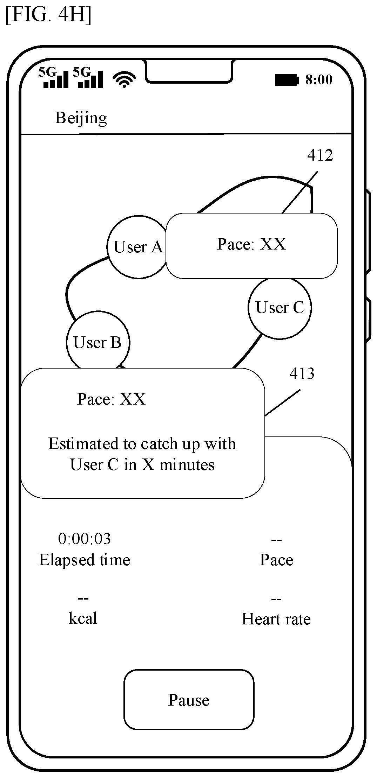

In a possible implementation, a catch-up target may be selected on a group running mode interface. As shown in FIG. 4G, in response to the operation performed by the user on the icon 409 of the user C, the mobile phone terminal displays a group running mode interface. The interface displays the prompt information 410. In addition to the running information of the user C, the prompt information 410 may further include a control 411, and the control 411 indicates that the user may set the user C as a catch-up target. In response to an operation performed by the user on the control 411, the mobile phone terminal may display a GUI shown in FIG. 4H.

As shown in FIG. 4H, in response to the operation performed by the user on the control 411, the mobile phone terminal displays a group running mode interface. The interface includes prompt information 412 and prompt information 413. The prompt information 412 indicates running information of the catch-up target, namely, the user C, for example, a pace of the user C. The prompt information 413 indicates running information of the local user of the mobile phone terminal, for example, a pace of the local user, and an estimated time of x minutes for the local user to catch up with the user C.

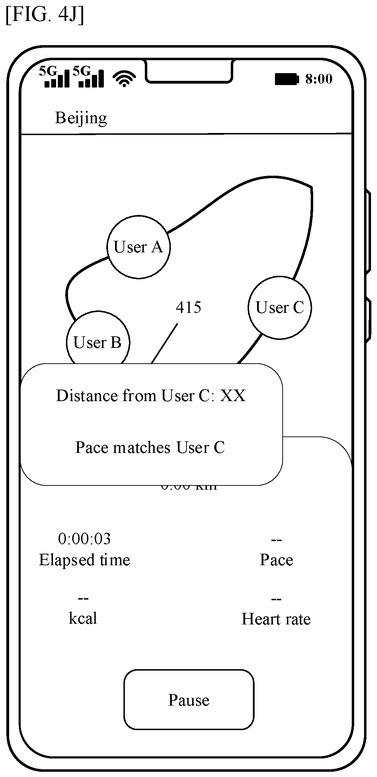

In another possible implementation, a pacer may be selected on a group running mode interface. As shown in FIG. 4I, in response to the operation performed by the user on the icon 409 of the user C, the mobile phone terminal displays a group running mode interface. The interface displays the prompt information 410. In addition to the running information of the user C, the prompt information 410 may further include a control 414, and the control 414 indicates that the user may set the user C as a pacer. In response to an operation performed by the user on the control 414, the mobile phone terminal may display a GUI shown in FIG. 4J. It should be noted that the control 411 and the control 414 may be simultaneously displayed in the prompt information 410, thereby allowing the user to choose whether to set the user C as a pacer or as a catch-up target.

As shown in FIG. 4J, in response to the operation performed by the user on the control 414, the mobile phone terminal displays a group running mode interface. The interface includes prompt information 415. The prompt information 415 is used to provide comparison information of exercise information such as a distance and a pace. For example, the prompt information 415 indicates a distance between the local user and the pacer (the user C), and may also indicate pace comparison information between the local user and the pacer (the user C). For example, the prompt information 415 indicates that the pace of the local user matches that of the user C, or indicates a difference between the pace of the local user and that of the user C. When the prompt information 415 indicates that the pace of the local user matches that of the user C, the local user may be advised to keep the current pace and continue exercising. When the prompt information 415 indicates that the pace of the local user is greater than that of the user C, the local user may be advised to reduce the pace, or the local user may be advised to reselect a more appropriate pacer. When the prompt information 415 indicates that the pace of the local user is less than that of the user C, the local user may be advised to increase the pace, or reselect a more appropriate pacer. That is, the user may adjust running rhythm of the user accordingly based on the pace comparison information.

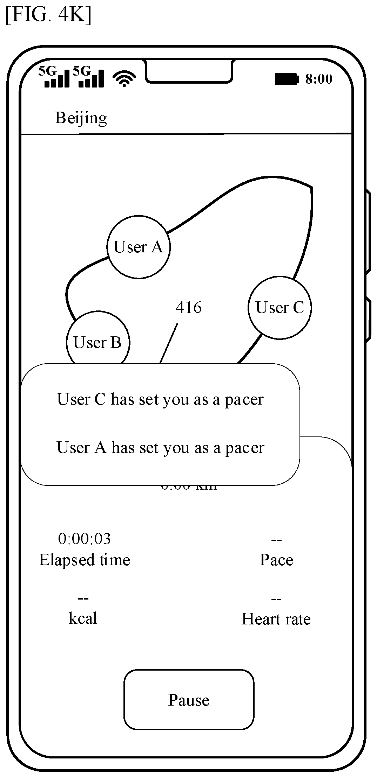

Certainly, the local user may also be set as a pacer by another user. When the local user is selected as a pacer, as shown in FIG. 4K, the mobile phone terminal displays a group running mode interface. Prompt information 416 is displayed on the interface. The prompt information 416 may indicate which users have set the local user as a pacer. For example, the prompt information 416 indicates that the user A has set the local user as a pacer, and that the user C has set the local user as a pacer. The user A and the user C are users who have joined a pacing group of the local user. The pacing group may include a pacer and other users, and the other users are users who have set the same user as their pacer. For example, in the GUI shown in FIG. 4K, the pacing group includes a pacer, namely, the user B, and the user A and the user C who have set the user B as the pacer.

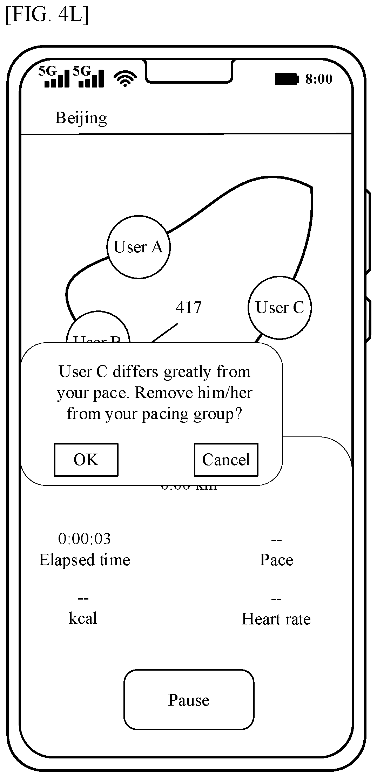

In a possible implementation, when a pace of a user in the pacing group differs greatly from that of the pacer, the pacer may remove the user from the pacing group. As shown in FIG. 4L, the mobile phone terminal displays a group running mode interface. The local user serves as a pacer. When the pace of the user C cannot keep up with that of the local user, prompt information 417 may be displayed. The prompt information 417 indicates that the pace of the user C differs greatly from that of the local user, and reminds the user whether to remove the user C from the pacing group of the local user. The prompt information 417 further includes an OK control and a cancel control. If the user taps the OK control, the user C is removed from the pacing group of the local user, and correspondingly, a device interface of the user C may display corresponding prompt information. Optionally, to ensure safety of the users during exercise, when the pace of the user C cannot keep up with that of the local user, the user C may be automatically removed from the pacing group of the local user, or a more appropriate user is recommended as a pacer for the user C.

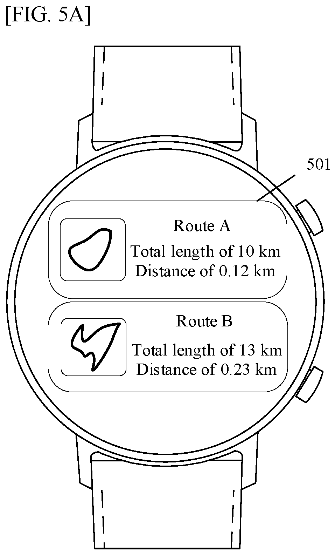

It should be noted that the electronic device that provides an exercise assistance function may alternatively be a smartwatch. With reference to FIG. 5A to FIG. 5M, the following shows a group of GUIs according to an embodiment of this application, and the GUIs may be displayed on a smartwatch. The smartwatch may provide more convenient interaction implementation for a user in an exercise scenario. The smartwatch is usually in a binding state with a mobile phone terminal, and the smartwatch may also carry a Health application. Therefore, content displayed on the smartwatch is basically the same as content displayed on the mobile phone terminal, and the content displayed on the smartwatch may be more simplified to facilitate a user operation. The following describes main interaction content displayed on the smartwatch.

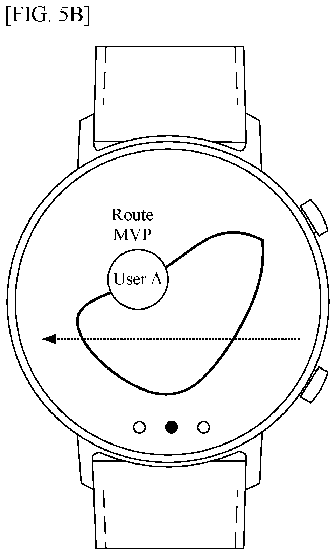

As shown in FIG. 5A, the smartwatch displays a running route interface. A plurality of route labels are displayed on the interface, and a plurality of routes are arranged in a specific order. For example, the plurality of route labels are arranged in order of distance from a current location of the user, and the plurality of route labels include a label of a route A and a label of a route B. Each route label displays basic information of the route. For example, the label of the route A indicates that a total length of the route A is 10 km and a distance from the current location of the user is 0.12 km. In response to an operation performed by the user on the label 501 of the route A, the smartwatch may display a GUI shown in FIG. 5B.

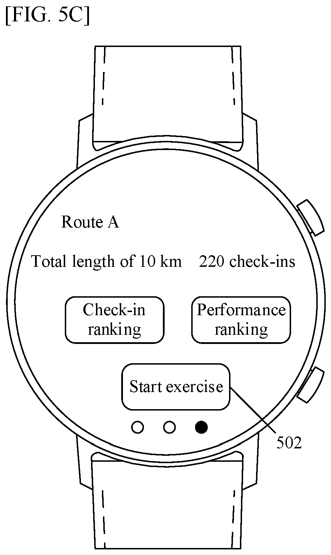

As shown in FIG. 5B, in response to the operation performed by the user on the route label 501, the smartwatch displays an interface of the route A. The interface displays the route A selected by the user, and may further display an MVP user of the route. For example, if a user A is displayed as the route MVP of the route A, it may indicate that the user A has the most check-ins on the route A, or the user A has the longest exercise time or the longest exercise distance along the route A, or the user A has the highest step frequency or the fastest pace on the route A. In response to a leftward swipe operation performed by the user in a direction indicated by a dashed arrow, the smartwatch may display a GUI shown in FIG. 5C. It should be noted that the swipe operation in this embodiment of this application is merely an example for description. This application does not impose any limitation on the direction of the swipe operation, nor does it impose any limitation on whether the swipe is performed with one finger, two fingers, or three fingers.

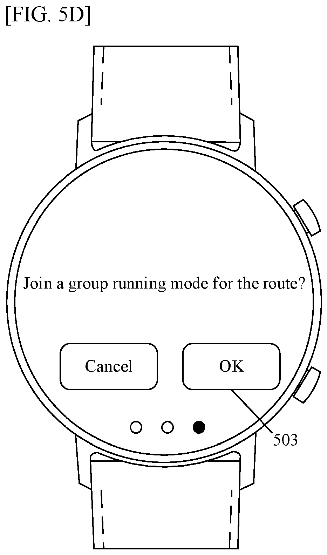

As shown in FIG. 5C, in response to the leftward swipe operation performed by the user, the smartwatch displays basic information of the route A. The basic information of the route A includes a route length, a quantity of check-in users, and the like. For example, the route A has a total length of 10 km and has a total of 220 check-ins. The interface further displays a check-in ranking control, a performance ranking control, and a start exercise control 502. In response to an operation performed by the user on the start control 502, the smartwatch may display a GUI shown in FIG. 5D. It should be noted that the operation for triggering the smartwatch to display the basic information about the route A may be a swipe operation performed by the user, or may be an operation in which the user taps a control or an icon. This is not limited in embodiments of this application. In the following description, information capable of being triggered for display via a swipe operation may also be triggered via an operation in which the user taps a control or an icon.

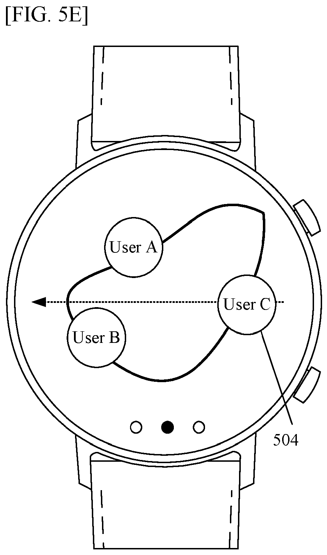

As shown in FIG. 5D, in response to the operation performed by the user on the start control 502, the smartwatch displays prompt information. The prompt information is used to prompt the user whether to join a group running mode for the route. The prompt information includes an OK control 503 and a cancel control. In response to an operation performed by the user on the OK control 503, the smartwatch may display a GUI shown in FIG. 5E.

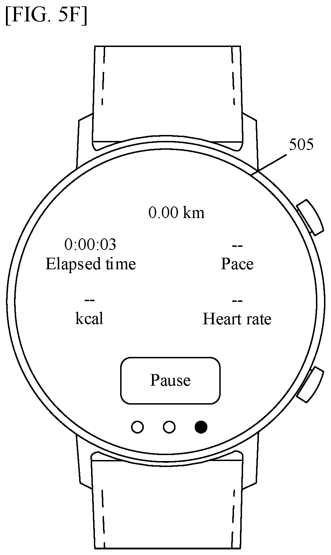

As shown in FIG. 5E, in response to the operation performed by the user on the OK control 503, the smartwatch displays a group running mode interface. This interface displays basic information of users who have joined the group running mode for the route A. For example, the users who have joined the group running mode for the route A include the user A, a user B, and a user C. The interface displays an icon of the user A, an icon of the user B, and an icon of the user C. A display position of the user icon shown in FIG. 5E may be updated with a change of a real-time location of the user. On the interface, in response to a leftward swipe operation performed by the user in a direction indicated by a dashed arrow, the smartwatch may display a GUI shown in FIG. 5F.

As shown in FIG. 5F, in response to the leftward swipe operation performed by the user, the smartwatch displays prompt information 505, and the prompt information 505 includes running information of a local user of the smartwatch, for example, current kilometers run, elapsed time, a pace, energy consumed (kilocalorie), and a heart rate.

Alternatively, the local user may view, on the group running mode interface shown in FIG. 5E, running information of other users who have joined the group running mode for A, where the running information may include real-time location information, a step frequency, a pace, and the like, to implement interactions such as setting a catch-up target or setting a pacer by the local user.

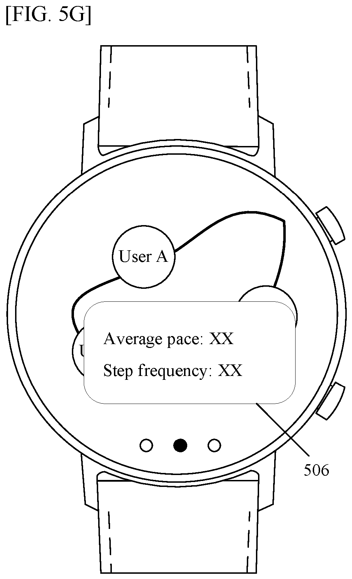

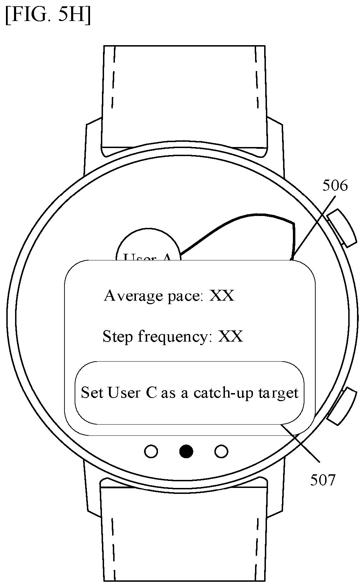

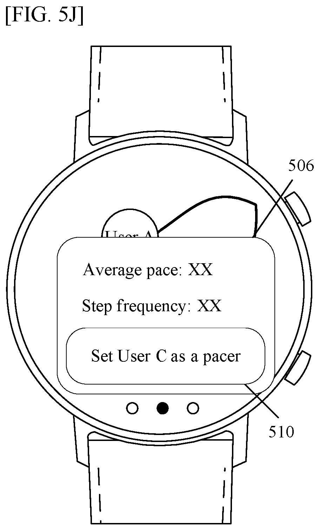

For example, in response to an operation performed by the user on the icon 504 of the user C shown in FIG. 5E, the smartwatch may display a GUI shown in FIG. 5G, or a GUI shown in FIG. 5H, or a GUI shown in FIG. 5J.

As shown in FIG. 5G, in response to the operation performed by the user on the icon 504 of the user C, the smartwatch may display a group running mode interface. On the group mode interface, by performing operations on different user icons, the user may obtain running information of a user corresponding to each user icon, and richer interaction scenarios may be achieved. For example, prompt information 506 is displayed on the interface, and the prompt information 506 indicates running information of the user C, for example, an average pace and a step frequency of the user C. Optionally, the prompt information 506 may further indicate relative information between the user C and the local user, for example, a distance between the user C and the local user, or a pace difference between the local user and the user C.

Further, richer interaction scenarios may be further achieved on the interface.

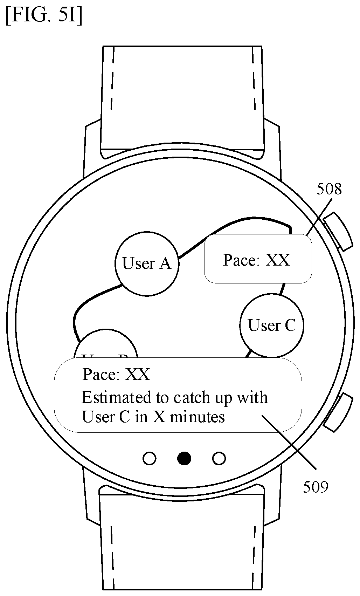

In a possible implementation, a catch-up target may be selected for the local user on a group running mode interface. As shown in FIG. 5H, in response to the operation performed by the user on the icon 504 of the user C, the smartwatch displays a group running mode interface. The interface displays the prompt information 506. In addition to the running information of the user C, the prompt information 506 may further include a control 507, and the control 507 indicates that the user may set the user C as a catch-up target. In response to an operation performed by the user on the control 507, the smartwatch may display a GUI shown in FIG. 5I.

As shown in FIG. 5I, in response to the operation performed by the user on the control 507, the smartwatch displays a group running mode interface. The interface includes prompt information 508 and prompt information 509. The prompt information 508 indicates running information of the catch-up target, namely, the user C, for example, a pace of the user C. The prompt information 509 indicates running information of the local user of the smartwatch, for example, a pace of the local user, and an estimated time of x minutes for the local user to catch up with the user C.

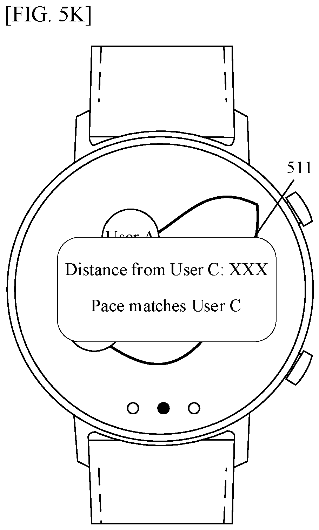

In another possible implementation, a pacer may be selected on a group running mode interface. As shown in FIG. 5J, in response to the operation performed by the user on the icon 504 of the user C, the smartwatch displays a group running mode interface. The interface displays the prompt information 506. In addition to the running information of the user C, the prompt information 506 may further include a control 510, and the control 510 indicates that the user may set the user C as a pacer. In response to an operation performed by the user on the control 510, the smartwatch may display a GUI shown in FIG. 5K. It should be noted that the control 507 and the control 510 may be simultaneously displayed in the prompt information 506, thereby allowing the user to choose whether to set the user C as a pacer or as a catch-up target.

As shown in FIG. 5K, in response to the operation performed by the user on the control 510, the smartwatch displays a group running mode interface. The interface includes prompt information 511. The prompt information 511 is used to provide comparison information of exercise information such as a distance and a pace. For example, the prompt information 511 indicates a distance between the local user and the pacer (the user C), and may also indicate pace comparison information between the local user and the pacer (the user C). For example, the prompt information 415 indicates that the pace of the local user matches that of the user C, or indicates a difference between the pace of the local user and that of the user C. When the prompt information 511 indicates that the pace of the local user matches that of the user C, the local user may be advised to keep the current pace and continue exercising. When the prompt information 511 indicates that the pace of the local user is greater than that of the user C, the local user may be advised to reduce the pace, or the local user may be advised to reselect a more appropriate pacer. When the prompt information 511 indicates that the pace of the local user is less than that of the user C, the local user may be advised to increase the pace, or reselect a more appropriate pacer. That is, the user may adjust running rhythm of the user accordingly based on the pace comparison information.

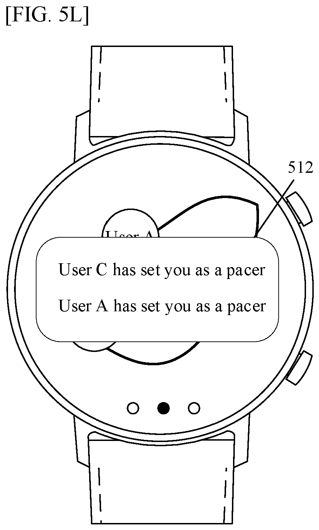

Certainly, the local user may also be set as a pacer by another user. When the local user is selected as a pacer, as shown in FIG. 5L, the smartwatch displays a group running mode interface. Prompt information 512 is displayed on the interface. The prompt information 512 may indicate which users have set the local user as a pacer. For example, the prompt information 512 indicates that the user A has set the local user as a pacer, and that the user C has set the local user as a pacer. The user A and the user C are users who have joined a pacing group of the local user. The pacing group may include a pacer and other users, and the other users are users who have set the same user as their pacer. For example, in the GUI shown in FIG. 5L, the pacing group includes a pacer, namely, the user B, and the user A and the user C who have set the user B as the pacer.

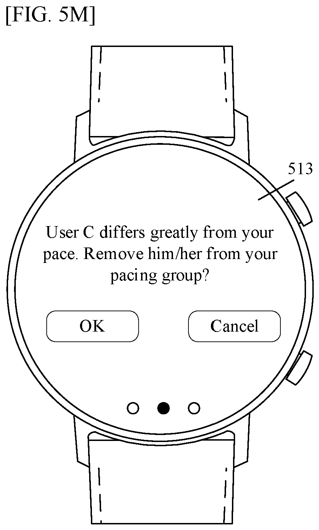

When a pace of a user in the pacing group differs greatly from that of the pacer, the pacer may remove the user from the pacing group. As shown in FIG. 5M, the smartwatch displays a group running mode interface. The local user serves as a pacer. When the pace of the user C cannot keep up with that of the local user, prompt information 513 may be displayed. The prompt information 513 indicates that the pace of the user C differs greatly from that of the local user, and reminds the user whether to remove the user C from the pacing group of the local user. Correspondingly, a device interface of the user C may display corresponding prompt information. Optionally, to ensure safety of the users during exercise, when the pace of the user C cannot keep up with that of the local user, the user C may be automatically removed from the pacing group of the local user, or a more appropriate user is recommended as a pacer for the user C.

Based on the foregoing solution, a user manually determines, via a display interface of an electronic device (for example, a mobile phone terminal or a smartwatch), a route for a group running mode that the user wants to join. In addition, after joining the group running mode for the route, the user may interact with other users who have joined the group running mode for the route, thereby increasing fun of the user during exercise.

Second, automatically determine a running route.

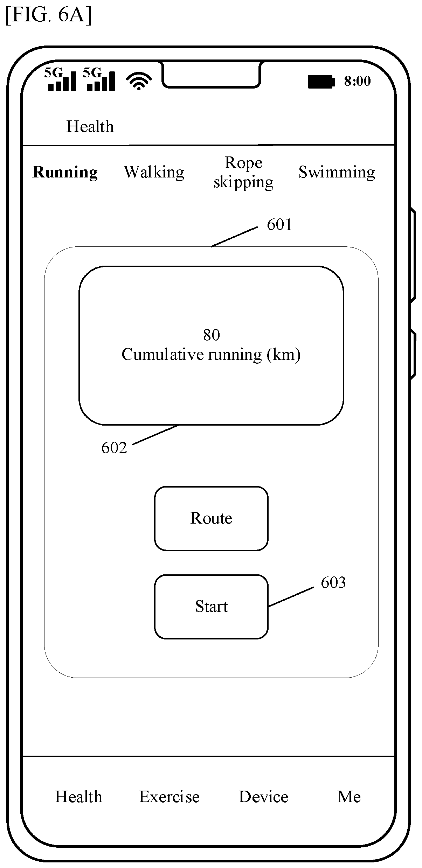

FIG. 6A to FIG. 6J show a group of GUIs according to an embodiment of this application, and describes a manner of automatically determining a running route.

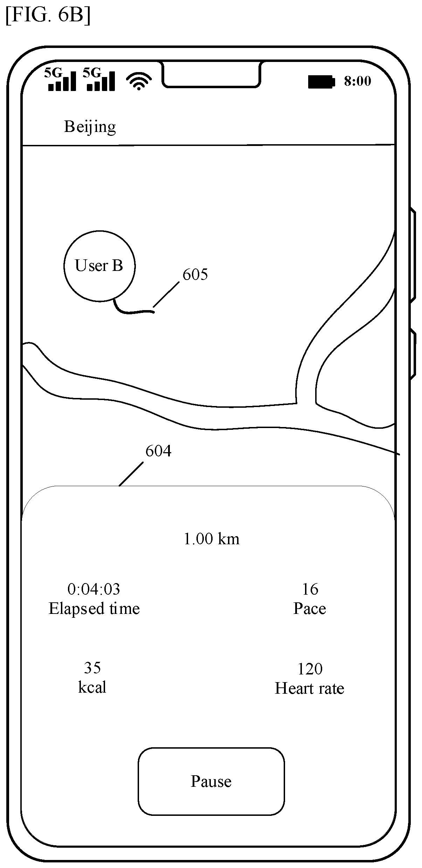

FIG. 6A shows a display interface of a Health app of a mobile phone terminal. The bottom of the display interface includes a plurality of controls, for example, a health control, an exercise control, a device control, and a Me control. The current display interface of the Health app corresponds to an interface displayed when the user taps the exercise control. The top of the display interface further includes a plurality of controls, for example, a running control, a walking control, a rope skipping control, and a swimming control. The display interface corresponds to an interface displayed when the user taps the running control. The display interface further includes prompt information 601, where the prompt information 601 includes cumulative running kilometer information 602, and further includes a route control and a start control. In response to an operation performed by the user on the start control 603, the mobile phone terminal may display a GUI shown in FIG. 6B.

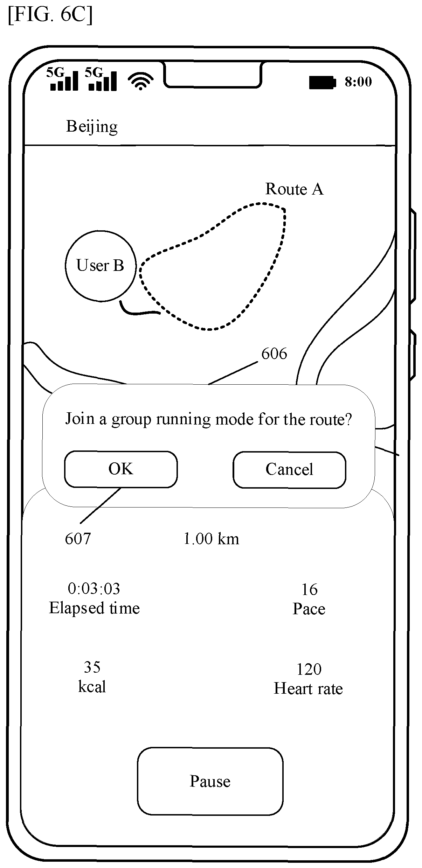

As shown in FIG. 6B, in response to the operation performed by the user on the start control 603, the mobile phone terminal displays a running interface. The interface displays prompt information 604, and the prompt information 604 includes current running information of a local user of the mobile phone terminal, for example, current kilometers run, elapsed time, a pace, energy consumed (kilocalorie), and a heart rate. The display interface further displays a route 605 that the local user has currently run. When the current kilometers run by the local user reach a specific distance threshold, the mobile phone terminal may display a GUI shown in FIG. 6C. The distance threshold is preset by the system, or may be customized by the user.

As shown in FIG. 6C, in response to the fact that the current kilometers run by the user reach the specific distance threshold, the mobile phone terminal displays a running interface. The interface displays prompt information 606, and the prompt information 606 is used to prompt the user whether to join a group running mode for a route A. The prompt information 606 includes an OK control and a cancel control. A dashed line portion of the interface indicates the route A. The route A is a target route that is automatically matched for the user.

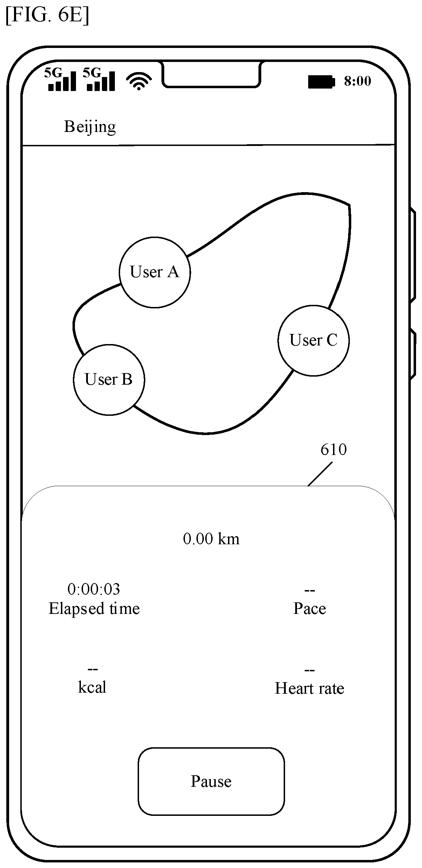

It may be understood that, when the current kilometers run by the user reach the specific distance threshold, the mobile phone terminal may match an appropriate target route based on a route that the user has currently run, and remind the user whether to join a group running mode for the target route. That is, when the mobile phone terminal matches a target route, the prompt information 606 may be displayed. In response to an operation performed by the user on an OK control 607, the mobile phone terminal may display a GUI shown in FIG. 6E.

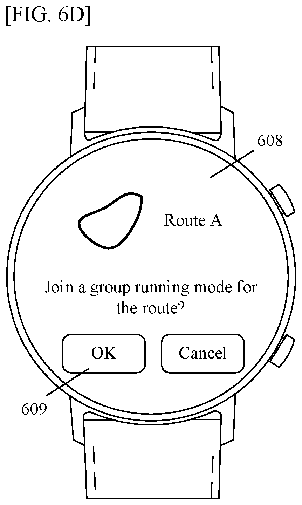

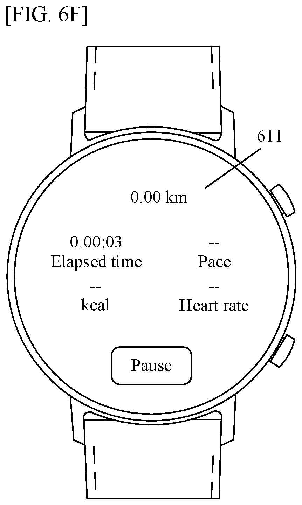

It should be noted that the electronic device that provides an exercise assistance function may alternatively be a smartwatch. As shown in FIG. 6D, in response to the fact that the current kilometers run by the user reach the specific distance threshold, the smartwatch displays prompt information 608, where the prompt information 608 is used to prompt the user whether to join the group running mode for the route A. That is, when the mobile phone terminal connected to the smartwatch matches the target route, the smartwatch may also synchronously display the prompt information 608 when the mobile phone terminal displays the prompt information 606. The smartwatch further displays an OK control and a cancel control. In response to an operation performed by the user on the OK control 609, the smartphone may display a GUI shown in FIG. 6F.

As shown in FIG. 6E, in response to the operation performed by the user on the OK control 607, the mobile phone terminal displays a group running mode interface. The interface displays basic information of users who have joined the group running mode for the route A. For example, the users who have joined the group running mode for the route A include a user A, a user B, and a user C. The interface displays an icon of the user A, an icon of the user B, and an icon of the user C. Prompt information 610 is further displayed on the group running mode interface, and the prompt information 610 includes current running information of the local user of the mobile phone terminal, for example, current kilometers run, elapsed time, a pace, energy consumed (kilocalorie), and a heart rate. A plurality of users who have joined the group running mode for the route A may perform simple interactions on an interface of the electronic device, in a manner similar to that described in FIG. 4E and FIG. 4F in the foregoing manner of manually determining a route. Details are not described herein again.

Similarly, in response to the operation performed by the user on the OK control 608, the smartwatch may display prompt information 611. The prompt information 611 may indicate current running information of the local user, for example, current kilometers run, elapsed time, a pace, energy consumed (kilocalorie), and a heart rate. That is, when the local user joins the group running mode, the smartwatch may synchronously display the prompt information with the mobile phone terminal.

In another possible implementation, the mobile phone terminal may match a plurality of appropriate routes based on the route that the user has currently run, and the user may select any route from the plurality of appropriate routes as the target route.

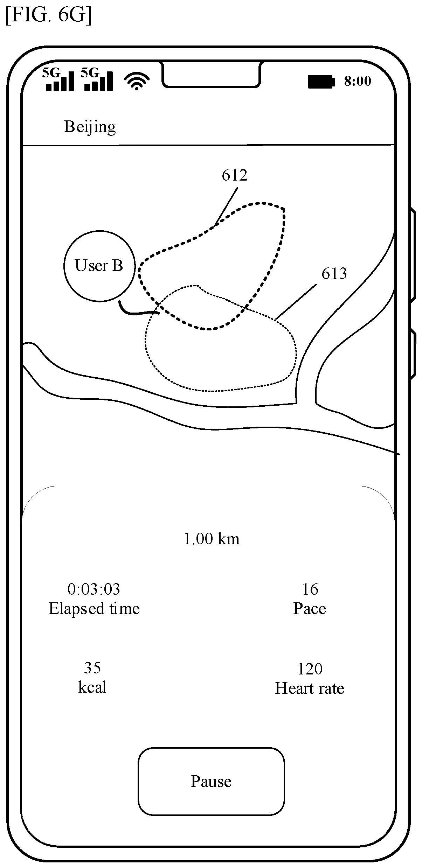

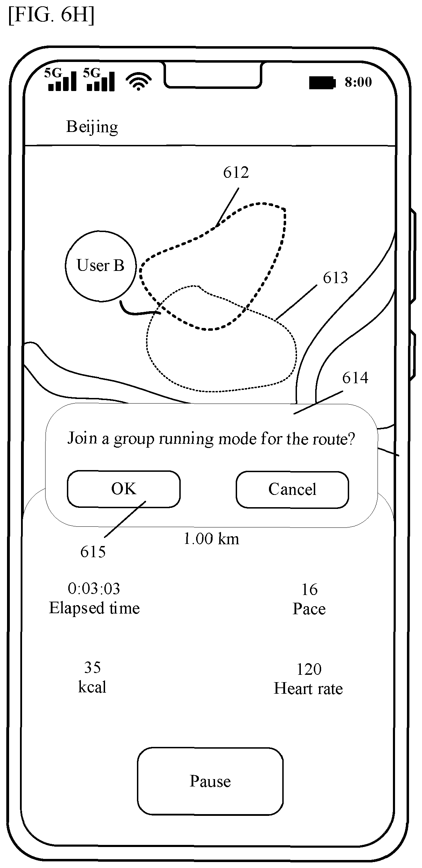

As shown in FIG. 6G, in response to the fact that the current kilometers run by the user reach the specific distance threshold, the mobile phone terminal displays a running interface. The interface displays icons of a plurality of to-be-selected routes, including a to-be-selected route icon 612 and a to-be-selected route icon 613. The to-be-selected routes are a plurality of appropriate routes matched by the mobile phone terminal for the user, and the user may select any route from the to-be-selected routes as the target route. In response to an operation performed by the user on the to-be-selected route icon 612, the mobile phone terminal may display a GUI shown in FIG. 6H.

As shown in FIG. 6H, in response to the operation performed by the user on the to-be-selected route icon 612, the mobile phone terminal displays a running interface. The interface displays prompt information 614, and the prompt information 614 is used to prompt the user whether to join a group running mode for the route. The prompt information 614 includes an OK control and a cancel control. In response to an operation performed by the user on the OK control 615, the mobile phone terminal may display a GUI shown in FIG. 6E, indicating that the user joins the group running mode for the route.

Certainly, this possible implementation may also be implemented on the smartwatch.

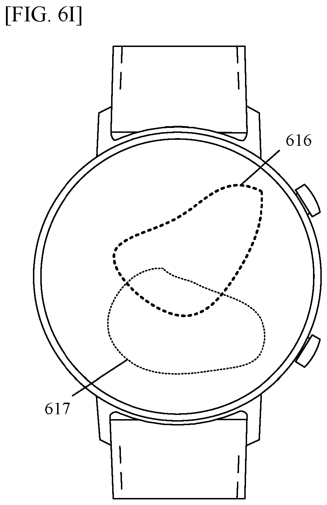

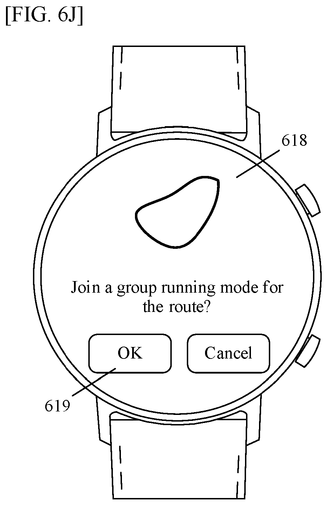

As shown in FIG. 6I, in response to the fact that the current kilometers run by the user reach the specific distance threshold, the smartwatch displays a running interface. The interface displays icons of a plurality of to-be-selected routes, including a to-be-selected route icon 616 and a to-be-selected route icon 617. The user may select any route from the plurality of to-be-selected routes as the target route. In response to an operation performed by the user on the to-be-selected route 616, the smartwatch may display a GUI shown in FIG. 6J.

As shown in FIG. 6J, in response to the operation performed by the user on the to-be-selected route icon 616, the smartwatch displays prompt information 618. The prompt information 618 is used to prompt the user whether to join a group running mode for the route. The prompt information 618 further includes an OK control and a cancel control. In response to an operation performed by the user on the OK control 619, the smartwatch may display a GUI shown in FIG. 6F, indicating that the user joins the group running mode for the route.

It should be noted that in the foregoing manner of automatically determining a running route, matching, by the mobile phone terminal, a target route based on an existing running route of the user is merely an example of determining the target route. A specific basis for determining the target route is not limited in embodiments of this application. The mobile phone terminal may alternatively match the target route based on a current location of the user.

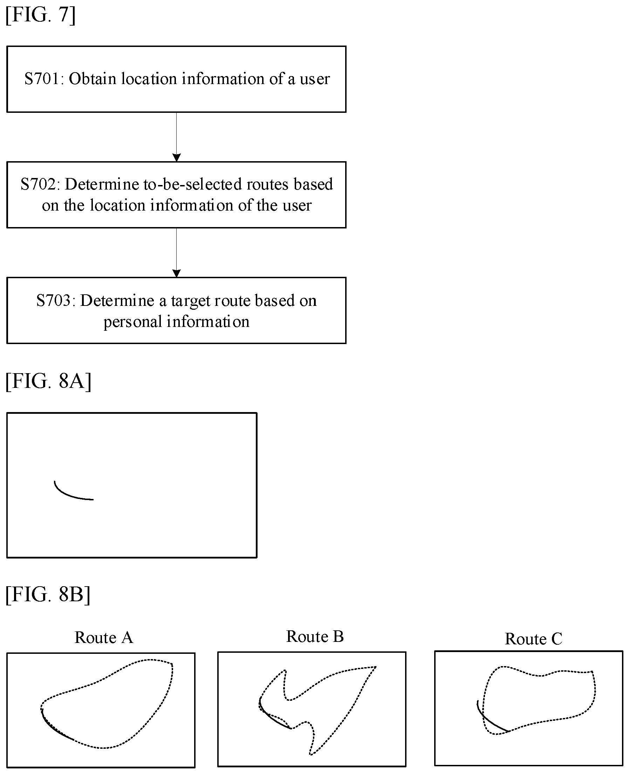

With reference to FIG. 7, the following specifically describes a method for automatically determining a running route in an embodiment of this application. The method may be applied to an electronic device that supports a route matching function, such as a mobile phone terminal.

S701: Obtain location information of a user.

For example, the location information of the user is obtained by using a global positioning system (global positioning system, GPS). The location information of the user may be a current location of the user, or may be a running route of the user within a time threshold, or may be a running route obtained when the user reaches a distance threshold. In the foregoing description of FIG. 6A to FIG. 6J, an example in which the location information of the user is the running route obtained when the user reaches the distance threshold is used for description.

S702: Determine to-be-selected routes based on the location information of the user.

It should be noted that the to-be-selected routes may be a plurality of routes near a current location of the user. The to-be-selected routes may be routes preset by the system, for example, the route A shown in FIG. 6C.

For example, manners of determining the to-be-selected routes based on the location information of the user include but are not limited to the following several manners.

Manner 1: The to-be-selected routes are determined based on the current location of the user.

For example, routes within a specific range from the current location of the user may be used as the to-be-selected routes.

Manner 2: The to-be-selected routes are determined based on a route on which the user runs within the time threshold.

For example, the time threshold is set to 2 minutes, and routes within a specific range of a running route of the user over the two minutes are used as the to-be-selected routes.

Manner 3: The to-be-selected routes are determined based on a running route obtained when the user reaches the distance threshold.

For example, the distance threshold is set to 1 km, a running route obtained when the user reaches 1 km is determined, and routes within a specific range of the route are used as the to-be-selected routes.

It should be noted that, in the foregoing three example manners of determining the to-be-selected routes, the to-be-selected routes are routes within a specific range of the current location of the user or the running route of the user. To determine more appropriate to-be-selected routes, the routes within a specific range may be further screened to select those with a high degree of overlap with the running route of the user as the to-be-selected routes. This manner of further screening the to-be-selected routes may be applicable to the foregoing Manner 2 and Manner 3.

For example, in Manner 2, based on the running route of the user over the two minutes, routes with a degree of overlap greater than a threshold with the running route of the user are selected from the routes within the specific range of the route as the to-be-selected routes. In Manner 3, based on the running route obtained when the user reaches one kilometer, routes with a degree of overlap greater than a threshold with the running route of the user are selected from the routes within the specific range of the route as the to-be-selected routes.

S703: Determine a target route based on personal information.

It should be noted that the target route may be determined from the plurality of to-be-selected routes based on the personal information, to serve as a route for a group running mode that the user may join.

For example, the target route is determined from the to-be-selected routes based on an exercise habit of the user. For example, the target route is determined based on a historical route of the user.

In a possible implementation, the target route is determined based on a route selection frequency of the user. For example, the to-be-selected routes include a route 1, a route 2, and a route 3. A frequency at which the user selects the route 1 as the target route is 5, a frequency at which the user selects the route 2 as the target route is 3, and a frequency at which the user selects the route 3 as the target route is 2. It is determined, based on the selection frequency of the user, that a route with a highest selection frequency is the route 1. Based on this, the route 1 may be determined as the target route.

In another possible implementation, the target route is determined based on a historical data pattern of the user. For example, the to-be-selected routes include a route 1, a route 2, and a route 3. The route 1 has a total length of 5 km, the route 2 has a total length of 10 km, and the route 3 has a total length of 15 km. The historical data pattern of the user may be that after the user exercises every five times, the running distance may be increased by 500 m, to achieve a purpose of enhancing exercise. If an exercise distance of the user increases to 10 km this time, the route 2 may be selected as the target route.

For example, the target route is determined from the to-be-selected routes based on a personal capability of the user.

It should be noted that the personal capability of the user may be represented by a plurality of parameters. For example, the plurality of parameters include a historical exercise state parameter of the user, exercise feedback information of the user, an exercise capability of the user, and the like. The historical exercise state parameter of the user includes a heart rate. The exercise feedback information of the user may be a post-exercise evaluation of a difficulty of each exercise session. The user may consider the exercise session as easy, difficult, or moderate in difficulty. The exercise capability of the user may be an evaluation by the Health application with respect to various aspects, including a training index, a recovery index, a running power index, and the like of the user. The personal capability of the user may alternatively be a target mode customized by the user. For example, the target mode includes a relaxed mode, an adaptive mode, a challenge mode, or the like. If the user selects the challenge mode, a route that exceeds a normal exercise distance of the user may be selected as the target route. If the user selects the adaptive mode, a route of a normal exercise distance of the user may be selected as the target route.

Further, if it is determined, based on the personal information, that there are a plurality of routes that meet the exercise habit and the personal capability of the user, a route with a highest degree of overlap with the running route of the user may be used as the target route with reference to Manner 3. Certainly, the plurality of routes that meet the exercise habit and the personal capability of the user may alternatively be displayed on the user interface, and the user autonomously selects any route as the target route.

It should be noted that, in addition to determining the to-be-selected routes based on the location information of the user, and determining the target route based on the personal information in the foregoing method 700, the to-be-selected routes may alternatively be determined in a manner described in the method for manually determining a target route. For example, if the user is currently in an unfamiliar environment, a nearest route may be recommended to the user as the target route. Alternatively, a route whose surface material is plastic is recommended, based on a preference of the user, to the user as the target route. In other words, the electronic device may determine the target route for the user based on a plurality of factors, and the plurality of factors may be combined for use. A basis for determining the target route is not limited in embodiments of this application.

For ease of understanding, with reference to FIG. 8A to FIG. 8E, the following describes a manner of determining the target route in the foregoing Manner 3.

FIG. 8A shows a running route of the user before joining the group running mode. For example, the running route is 1 km. The running route is an actual running route before the user joins a group running mode for a target route.

FIG. 8B shows three to-be-selected routes, including a route A, a route B, and a route C. A distance between the three to-be-selected routes and the actual running route of the user before joining the group running mode, as shown in FIG. 8A, is less than or equal to a preset threshold. In other words, there are three to-be-selected routes within a preset threshold range around the actual running route of the user. The three to-be-selected routes may be displayed on the user interface of the electronic device as prompt information, and the finally determined target route may also be displayed. In a possible implementation, the final target route may be determined based on a degree of overlap between the running route of the user and the to-be-selected routes. For example, if the route A has a highest degree of overlap with the running route of the user, the route A may be determined as the target route. It should be noted that, the basis for determining the target route may alternatively be the personal information, the road surface condition, or the like described above. Details are not described herein again.

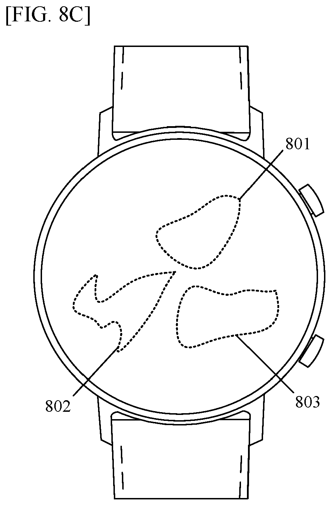

FIG. 8C shows a running interface of the smartwatch. A plurality of to-be-selected routes are displayed on the interface, including a to-be-selected route 801, a to-be-selected route 802, and a to-be-selected route 803. The user may select any of the routes as a target route and join a group running mode for the target route. In this implementation, the plurality of to-be-selected routes may be simultaneously displayed on the user interface of the electronic device, so that the user autonomously selects the target route.

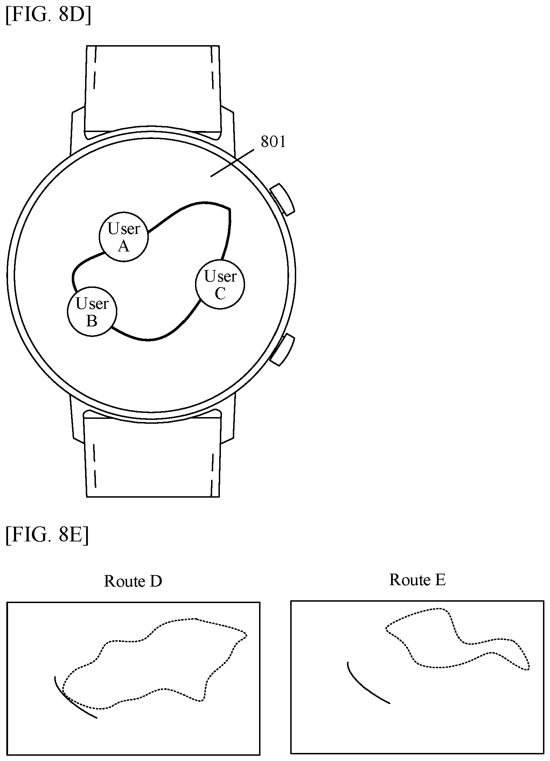

Alternatively, FIG. 8D shows a group running mode interface of the smartwatch, and a target route is directly displayed on the interface. For example, the to-be-selected target 801 is determined as the target route. The interface may further display icons of users who have joined a group running mode for the target route, for example, an icon of a user A, an icon of a user B, and an icon of a user C. In this implementation, the plurality of to-be-selected routes may not be simultaneously displayed on the user interface of the electronic device, so that the user can determine the target route without perception.

In addition, FIG. 8E shows two to-be-selected routes, including a route D and a route E. If only the route D and the route E exist within a range in which a distance from the running route obtained when the user reaches 1 km is less than or equal to the preset threshold, and a degree of overlap between the two routes and the running route of the user is less than the threshold, a route closest to the running route of the user is selected as the target route. For example, if a distance between the route D and the center of the running route of the user is less than that between the route D and the center of the running route of the user, the route D is determined as the target route.

It may be learned that the target route may be comprehensively determined based on factors such as a distance from the user, a degree of overlap with the running route of the user, and the personal information of the user.

It should be noted that, when the user joins a group running mode for a route and may possibly deviate from the route during running, the electronic device may guide the user to return to the correct route through voice or display interaction.

### Scenario 2: Running route correction

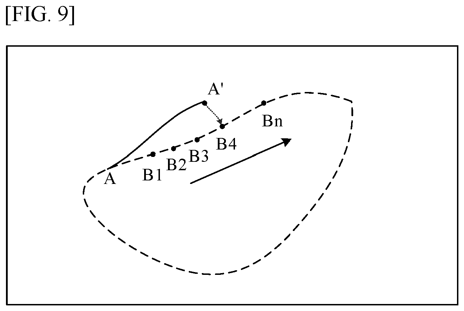

FIG. 9 is a diagram of a method for correcting a running route according to an embodiment of this application. A closed region enclosed by a dashed line in FIG. 9 is a route of a group running mode that the user has joined, and may be understood as a correct route. The user should run along the route shown by the dashed line. A solid arrow indicates a running direction of the user. The user deviates from the route during running, starting from a point A on the route and reaching a point A'. After the user deviates by a specific distance, a plurality of location points are generated on the correct route near the point A', for example, B1, B2, B3, B4, ..., and Bn. A plurality of return routes are formed between the point A' and the foregoing plurality of location points. The user may return to the correct route by taking any one of the return routes. The electronic device may select a most appropriate location point from the foregoing plurality of location points based on a plurality of factors as a target point for guiding the user to return to the correct route. For example, actual paths between A' and B1, B2, B3, B4, ..., and Bn are considered, and actual distances of the plurality of return routes are calculated. By comparison, it is determined that the return routes between A' and B3 and the return route between A' and B4 have short actual distances. In this case, considering that the solid arrow indicates the running direction of the user, if the user chooses to return to the correct line along the return route between A' and B3, there is a specific degree of backtracking. If the user chooses to return to the correct line along the return route between A' and B4, the running route of the user is approximately consistent with the correct running direction, and there is no backtracking. Therefore, the point B4 may be determined as the target point, and the user may be guided from the current location at the point A' to run along the route and in the direction indicated by the arrow toward the point B4, to return to the correct route. It should be noted that the actual distances between A' and B1, B2, B3, B4, ..., and Bn are related to an actual road condition, and are not simple straight-line distances between two location points.

In a possible implementation, when the user deviates from the route, the user may be guided to select a new target route, or a new target route may be matched for the user.

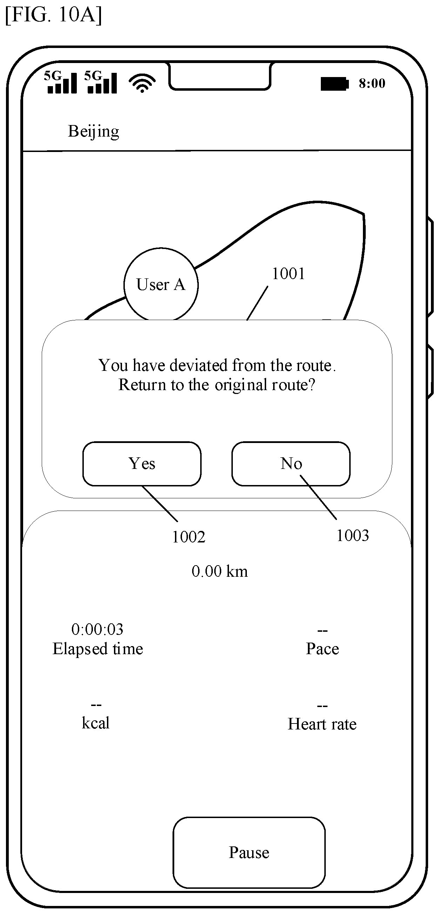

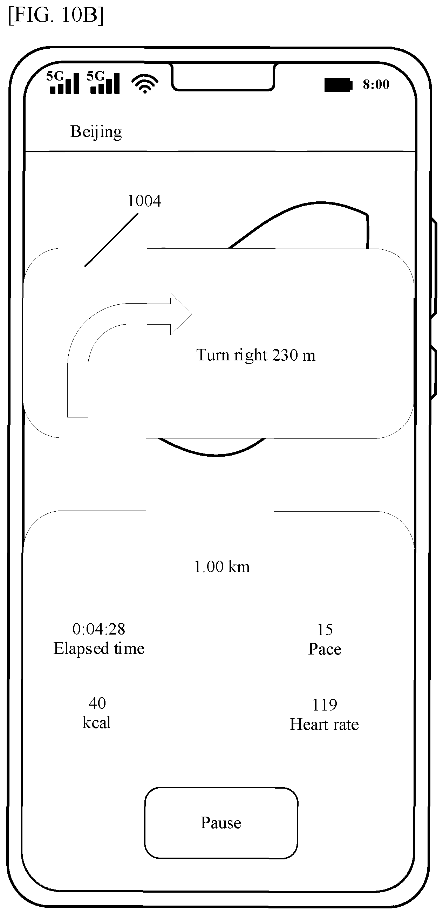

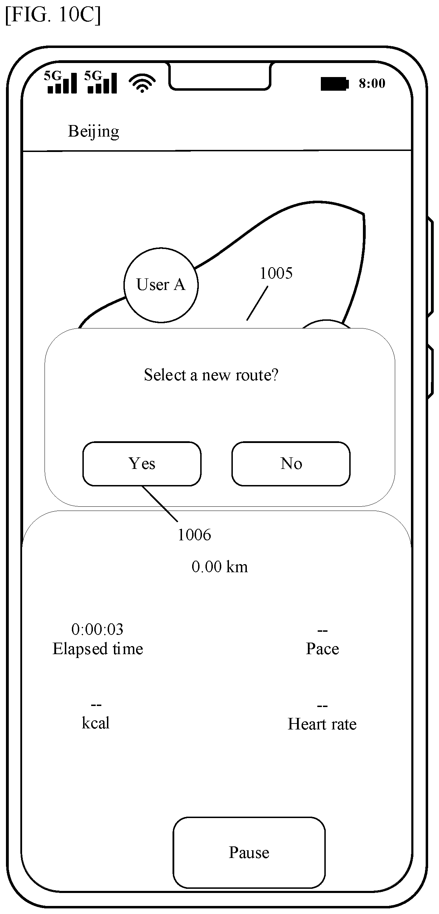

It should be noted that, when the user deviates from the route, the electronic device may remind the user whether guidance is needed to return to the original route, or directly guide the user to return to the original route, or remind the user whether to switch to a new route, or directly enter a free exercise mode, or the like. The following describes the process in detail with reference to FIG. 10A to FIG. 10F. It should be noted that the plurality of feedback manners of the electronic device in response to a deviation of the user from the route may be implemented independently or simultaneously. For example, when the user deviates from the route, the electronic device may directly guide the user to return to the original route, as shown in FIG. 10B, and directly display navigation information. For another example, when the user deviates from the route, the electronic device reminds the user whether guidance is needed to return to the original route. If the user chooses not to return to the original route, the electronic device may select a new route for the user. For another example, when the user deviates from the route, as shown in FIG. 10C, the electronic device directly reminds the user whether to switch to a new route.

As shown in FIG. 10A, the mobile phone terminal displays a group running mode interface, indicating that the user joins the group running mode for the route. After the user deviates from the route by a specific distance, prompt information 1001 is displayed on the interface, and the prompt information 1001 is used to prompt the user whether to return to the original route. The prompt information 1001 further includes a "Yes" control 1002 and a "No" control 1003. In response to an operation performed by the user on the "Yes" control 1002, the mobile phone terminal may display a GUI shown in FIG. 10B. In response to an operation performed by the user on the "No" control 1003, the mobile phone terminal may display a GUI shown in FIG. 10C, to remind the user whether to switch to a new route. Alternatively, in response to an operation performed by the user on the "No" control 1003, the mobile phone terminal switches to a free exercise mode. In the free exercise mode, a running route of the user is not limited.

As shown in FIG. 10B, in response to the operation performed by the user on the "Yes" control 1002, the mobile phone terminal displays a group running mode interface. The interface includes prompt information 1004, and the prompt information 1004 may be navigation information and is used to guide the user to return to the original route from the current location.

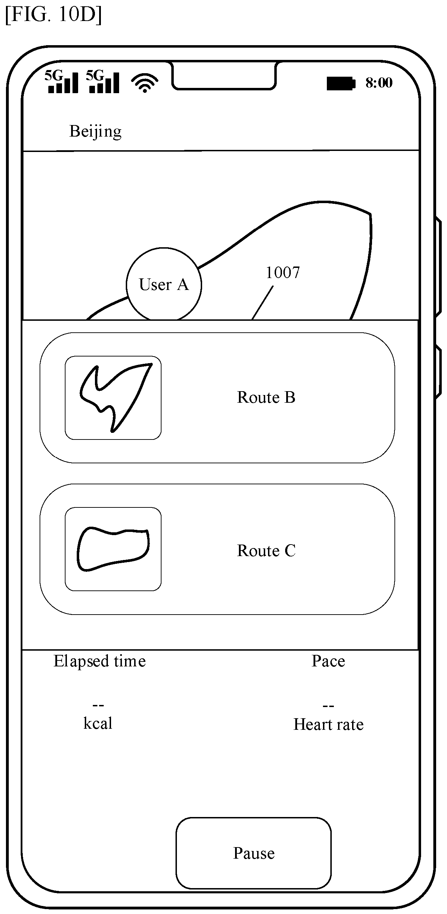

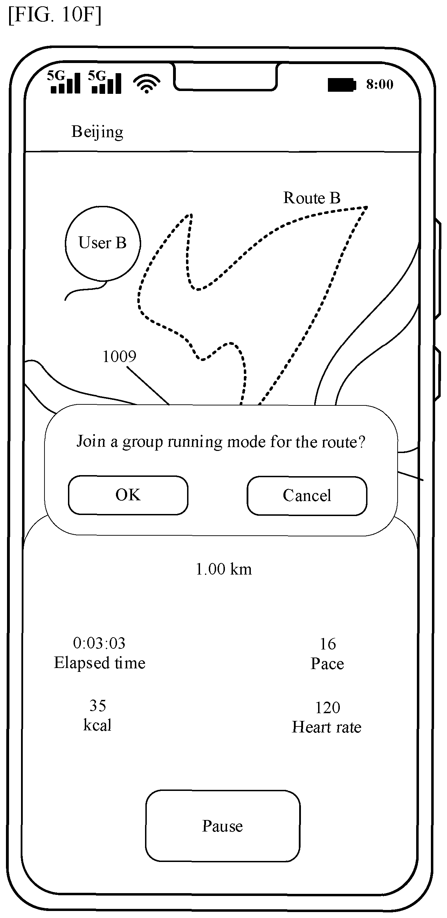

As shown in FIG. 10C, in response to the operation performed by the user on the "No" control 1003, the mobile phone terminal displays a group running mode interface. The interface displays prompt information 1005, and the prompt information 1005 is used to prompt the user whether to select a new route. The prompt information 1005 further includes a "Yes" control 1006 and a "No" control. In response to an operation performed by the user on the "Yes" control 1006, the mobile phone terminal may display a GUI shown in FIG. 10D, or display a GUI shown in FIG. 10F. In response to an operation performed by the user on the "No" control, the mobile phone terminal switches to the free exercise mode.

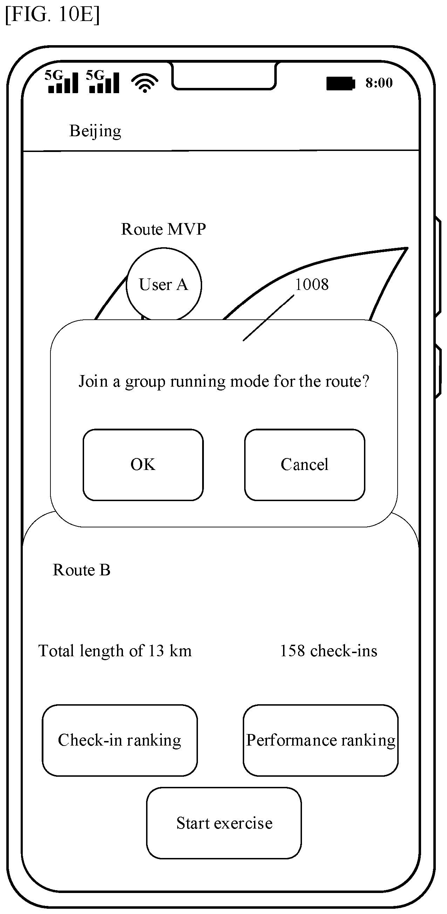

In an implementation, the user manually selects a new route. As shown in FIG. 10D, in response to the operation performed by the user on the "Yes" control 1006, the mobile phone terminal displays a group running mode interface. The interface displays prompt information 1007, and the prompt information 1007 includes a label of a to-be-selected route B and a label of a to-be-selected route C. In response to an operation performed by the user on the label of the to-be-selected route B, the mobile phone terminal may display a GUI shown in FIG. 10E.

As shown in FIG. 10E, in response to the operation performed by the user on the label of the to-be-selected route B, the mobile phone terminal displays an interface of the route B. The interface displays prompt information 1008, and the prompt information 1008 is used to prompt the user whether to join a group running mode for the route. The prompt information 1008 includes an OK control and a cancel control. When the user taps the OK control, the user may switch to the group running mode for the route B.

In another implementation, a new route is automatically matched for the user. In response to the operation performed by the user on the "Yes" control 1006, as shown in FIG. 10F, the mobile phone terminal displays an interface of the route B. Optionally, the interface displays prompt information 1009, and the prompt information 1009 is used to prompt the user whether to join a group running mode for the route. The prompt information 1009 includes an OK control and a cancel control. When the user taps the OK control, the user may switch to the group running mode for the route B.

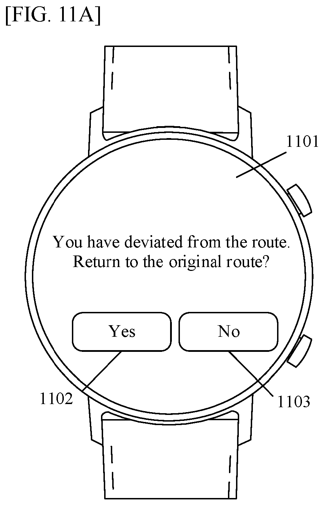

Certainly, the foregoing interaction process may also be implemented on the smartwatch. FIG. 11A to FIG. 11E show a group of GUIs on the smartwatch.

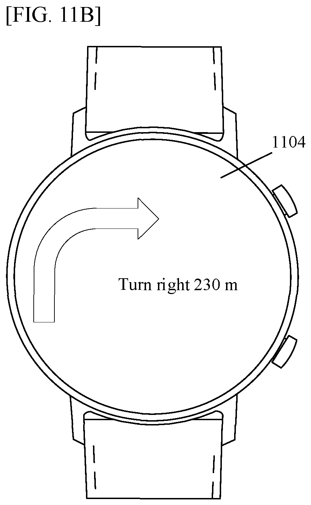

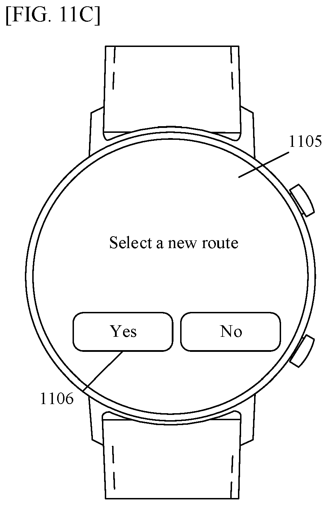

As shown in FIG. 11A, when the user deviates from the original route by a specific distance, the smartwatch displays prompt information 1101, where the prompt information 1101 is used to prompt the user whether to return to the original route. The prompt information 1101 includes a "Yes" control 1102 and a "No" control 1103. In response to an operation performed by the user on the "Yes" control 1102, the smartwatch may display a GUI shown in FIG. 11B. In response to an operation performed by the user on the "No" control 1103, the smartwatch may display a GUI shown in FIG. 11C, to remind the user whether to switch to a new route. Alternatively, in response to an operation performed by the user on the "No" control 1103, the smartwatch switches to a free exercise mode. In the free exercise mode, a running route of the user is not limited.

As shown in FIG. 11B, in response to the operation performed by the user on the "Yes" control 1102, the smartwatch displays prompt information 1104. The prompt information 1104 may be navigation information and is used to prompt the user to return to the original route from the current location.

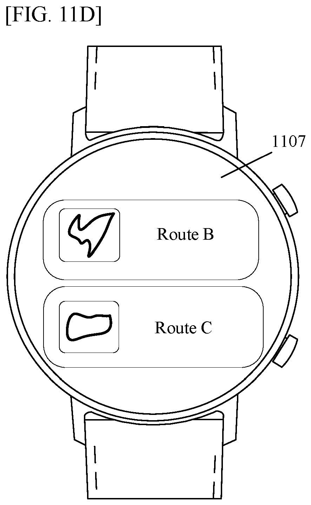

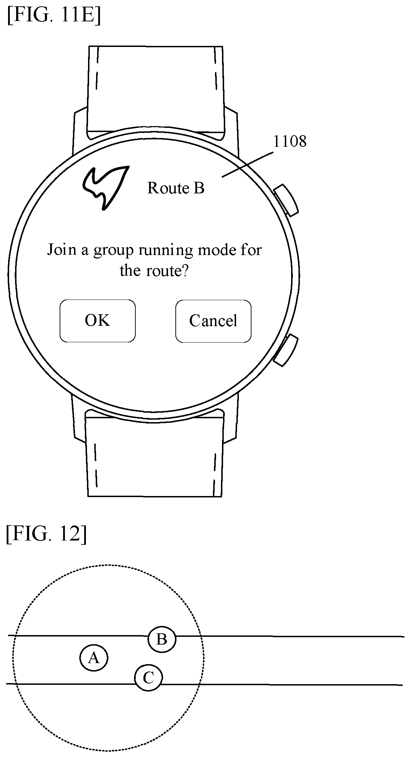

As shown in FIG. 11C, in response to the operation performed by the user on the "No" control 1103, the smartwatch displays prompt information 1105, where the prompt information 1105 is used to prompt the user whether to select a new route. The prompt information 1105 further includes a "Yes" control 1106 and a "No" control. In response to an operation performed by the user on the "Yes" control 1106, the smartwatch may display a GUI shown in FIG. 11D, or directly display a GUI shown in FIG. 11E. In response to an operation performed by the user on the "No" control, the smartwatch switches to the free exercise mode.

In an implementation, the user manually selects a new route. As shown in FIG. 11D, in response to the operation performed by the user on the "Yes" control 1106, the smartwatch displays prompt information 1107, where the prompt information 1107 includes a label of a to-be-selected route B and a label of a to-be-selected route C. In response to an operation performed by the user on the label of the to-be-selected route B, the smartwatch may display a GUI shown in FIG. 11E.

In another implementation, a new route is automatically matched for the user. As shown in FIG. 11E, in response to the operation performed by the user on the "Yes" control, the smartwatch displays prompt information 1108, where the prompt information 1108 is used to prompt the user whether to join a group running mode for the route. The prompt information 1108 includes an OK control and a cancel control. When the user taps the OK control, the user may switch to the group running mode for the route B. Certainly, the prompt information 1108 shown in FIG. 11E may alternatively be triggered and displayed by manually selecting a route.

It should be noted that a specific manner in which the electronic device guides the user to return to the correct route is not limited in embodiments of this application. The electronic device may guide the user to return to the correct route by displaying prompt information, or may guide the user to return to the correct route by playing a prompt voice.

It should be noted that, in the group running mode, a user with a weak GPS signal cannot obtain accurate location information. In this case, location information of other surrounding users with a strong GPS signal may be used to assist the user with a weak signal in positioning.

### Scenario 3: Assisted positioning

FIG. 12 is a diagram of an assisted positioning method according to an embodiment of this application. In a group running mode for a same route, a user A, a user B, and a user C are included. A GPS signal of the user A is weak, and GPS signals of the user B and the user C around the user A are strong. Therefore, it is difficult to obtain accurate location information of the user A by using the GPS signal of the user A, and the user B and the user C can accurately determine location information of the user B and the user C by using their own GPS signals.

To better determine the location information of the user A, in a period of time before the GPS signal of the user A is weak, exercise information of a plurality of users with strong GPS signals around the user A is recorded, for example, a step frequency and an inertial measurement unit (inertial measurement unit, IMU) relative velocity. From the plurality of users around the user A, users whose step frequency and IMU relative velocity remain unchanged are selected as reference users (for example, the user B and the user C). When the GPS signal of the user A is weak, a GPS of the user A cannot accurately determine the location information of the user A. If the user A, the user B, and the user C can communicate with each other normally, the user A may receive location information from the user B and the user C, and calculate the location information of the user A based on the location information of the user B and the user C.

It should be noted that the group running mode provided in embodiments of this application may further include a normal mode and a race mode. The normal mode may be understood as a mode in which a plurality of users run on a same route within a period of time, and start time, start locations, and running targets of the plurality of users may all be different. The race mode may be understood as a mode in which a plurality of users run on a same route, and start time, start locations, and running targets of the plurality of users are the same. In different modes, the user can perform simple interactions via the electronic device.

### Scenario 4: Interaction between different modes

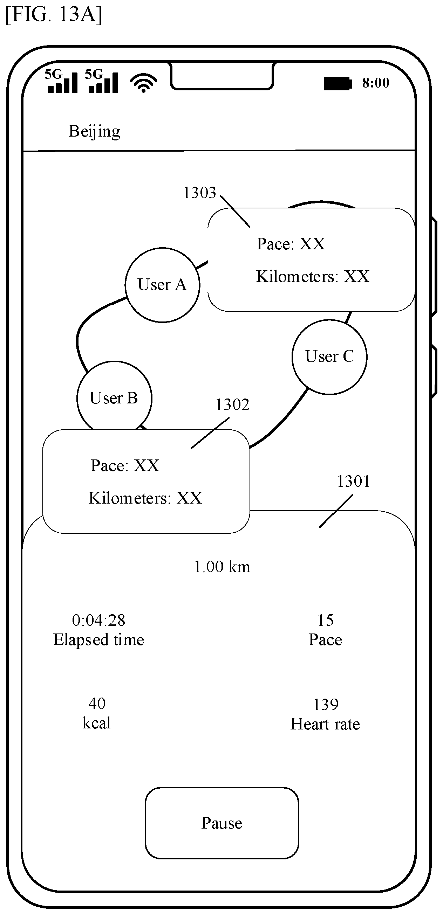

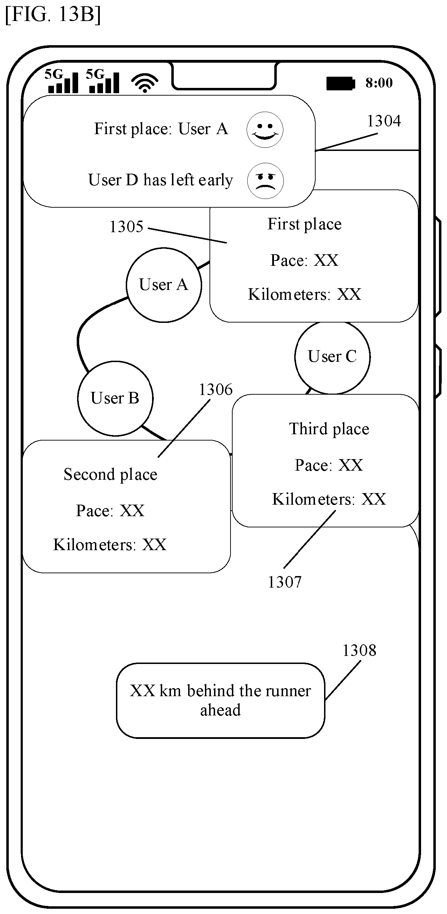

FIG. 13A and FIG. 13B show a group of GUIs according to an embodiment of this application, and describes an interaction manner in a group running mode.

In an implementation, an electronic device may implement interaction in a normal mode. FIG. 13A shows a group running mode interface of a mobile phone terminal. The interface displays basic information of users who have joined a group running mode for a same route. For example, the users who have joined the group running mode for the route include a user A, a user B, and a user C. The interface displays an icon of the user A, an icon of the user B, and an icon of the user C. Prompt information 1301 is further displayed on the group running mode interface, and the prompt information 1301 includes current running information of a local user of the mobile phone terminal, for example, current kilometers run, elapsed time, a pace, energy consumed (kilocalorie), and a heart rate. The interface further displays prompt information 1302 and prompt information 1303. The prompt information 1302 includes running information of the user B, for example, a pace and kilometers run by the user B. The prompt information 1303 includes running information of the user C, for example, a pace and kilometers run by the user C. It should be noted that FIG. 13A shows an interaction manner of the users in the normal mode. In the normal mode, running information of users who have joined a group running mode for a same route may be obtained via a screen of the electronic device, or one of the users may be set as a catch-up user, for example, as shown in FIG. 4E and FIG. 4F.

In another implementation, the electronic device may implement interaction in a race mode. It should be noted that the user may create a race or join an existing race. In a scenario in which the user creates a race, the user sets, in a Health application, a route of the race, users joining the race, whether to allow other users to join the race at any time, whether joining the race by other users requires permission from the local user, and race parameters (for example, elapsed time for the same distance, a distance run in the same elapsed time, and a pace), and the like. In a scenario in which the user joins an existing race, the user can join a race created by another user. A plurality of users belonging to the same race team can interact with each other.

FIG. 13B shows a group running mode interface of the mobile phone terminal. The interface displays basic information of users who have joined a group running mode for a same route. For example, the users who have joined the group running mode for the route include a user A, a user B, and a user C. The interface displays an icon of the user A, an icon of the user B, and an icon of the user C. FIG. 13B shows an interaction manner of the users in the race mode. In race mode, related information about the race may be displayed. The related information about the race includes ranking information, participant change information, exercise parameters of participants, comparison information of the exercise parameters, and the like. A list of users ranked based on a running distance, a list of users ranked based on a pace, or a list of users ranked based on running duration may be obtained by using the ranking information. Information of a user who joins the race in real time or information of a user who withdraws from the race in real time may be obtained by using the participant change information. Exercise parameters such as a pace, kilometers run, and running duration of each participant may be obtained by using the exercise parameters of the participants. Comparison information of exercise parameters between the local user and other participants may be obtained by using the comparison information of exercise parameters, which facilitates the user in learning about a gap between the local user and the other participants.

For example, the interface displays prompt information 1304, prompt information 1305, prompt information 1306, prompt information 1307, and prompt information 1308. The prompt information 1304 indicates ranking information and participant change information. For example, the prompt information 1304 indicates that the current first place is the user A, and further indicates that the user D has left the race early, or further indicates that a new user has joined the race or requests to join the race. The prompt information 1305, the prompt information 1306, and the prompt information 1307 indicate exercise parameters of the participants, including a pace, kilometers run, and the like of the participants. The prompt information 1305 is displayed near the icon of the user A, and indicates that the current first place is the user A. The prompt information 1305 displays a pace and kilometers run by the user A. The prompt information 1306 is displayed near the icon of the user B, and indicates that the current second place is the user B. The prompt information 1306 displays a pace and kilometers run by the user B. The prompt information 1307 is displayed near the icon of the user C, and indicates that the current third place is the user C. The prompt information 1307 displays a pace and kilometers run by the user C. The prompt information 1308 may be comparison information of an exercise parameter, and indicates comparison information of an exercise parameter between the local user and another participant. The specific prompt information 1308 displayed may vary depending on the specific information being compared. For example, when the information being compared is kilometers run, the prompt information 1308 indicates kilometers from the local user of the terminal device to the runner ahead. When the information being compared is running duration, the prompt information 1308 may indicate a time difference in running duration between the local user and the previous user. When the information being compared is a pace, the prompt information 1308 may indicate a pace difference between the local user and the previous user. Certainly, the prompt information 1308 may indicate not only comparison information of exercise parameters between the local user and the previous user, but also comparison information of exercise parameters between the local user and the first-place user.

It may be learned that interaction manners in the normal mode and the race mode may be different, so that in different modes, a user requirement is adapted in a more personalized manner, thereby providing more vivid interaction experience for the user.

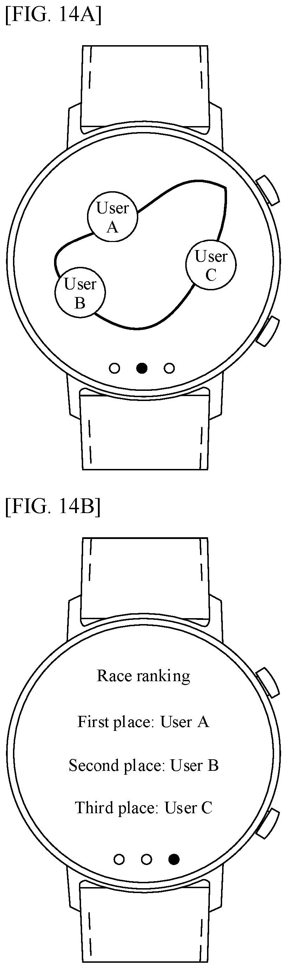

Certainly, the foregoing interaction process may also be implemented on a smartwatch. FIG. 14A to FIG. 14D show a group of GUIs on the smartwatch.

FIG. 14A shows a group running mode interface of the smartwatch. When the user is in the race mode, in response to a leftward swipe operation performed by the user on the interface, the smartwatch may display a GUI shown in FIG. 14B.

As shown in FIG. 14B, in response to the leftward swipe operation performed by the user, the smartwatch displays a race ranking interface. The interface displays ranking information. The ranking information may include a list of users ranked based on exercise parameters such as a distance run, running duration, and a pace. The interface shows that the first place is the user A, the second place is the user B, and the third place is the user C.

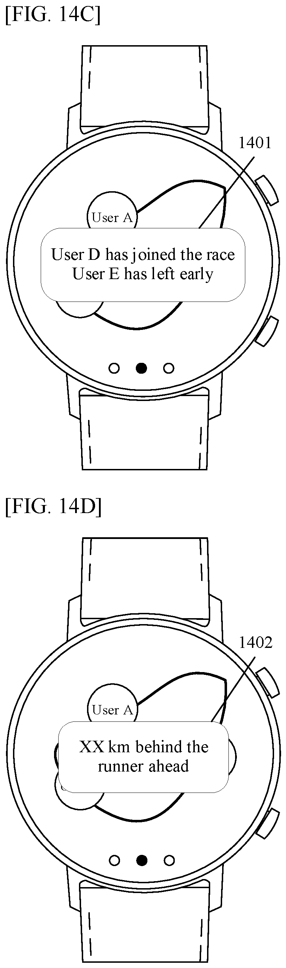

As shown in FIG. 14C, when a participant change occurs, the group running mode interface of the smartwatch displays prompt information 1401. The prompt information 1401 indicates the participant change, and includes information of a user who joins the race in real time or information of a user who withdraws from the race in real time. For example, the prompt information 1401 indicates that a user D has joined the race, and that a user E has left the race early.

As shown in FIG. 14D, the group running mode interface of the smartwatch periodically displays prompt information 1402, and the prompt information 1402 indicates comparison information of exercise parameters. The comparison information of exercise parameters facilitates the local user in learning out a gap between the local user and other participants. For example, the prompt information 1402 indicates kilometers from the local user to the runner ahead. Certainly, the prompt information 1402 may not only be periodically displayed on the user interface, but also be triggered to display by a user operation. This is not limited in this application.

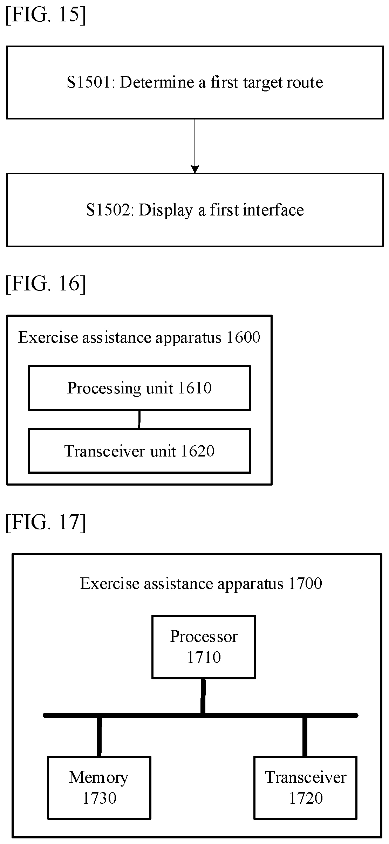

FIG. 15 shows an exercise assistance method according to an embodiment of this application. The method may be applied to an electronic device that supports an exercise assistance function, such as a mobile phone terminal. The following describes a method 1500 by using an example in which the method is applied to a first electronic device (a mobile phone terminal).

S1501: Determine a first target route.

It should be noted that before a user joins a group running mode, a route needs to be determined as a target route. Manners of determining the target route include determining manually and determining automatically.

In an implementation, the user manually determines the first target route.

For example, a second interface is displayed, where the second interface displays a label of at least one to-be-selected route, and the label of the at least one to-be-selected route includes a label of a first route; and an operation performed by the user on the label of the first route is received, and the first route is determined as the first target route.

It should be noted that the second interface may be the running interface shown in FIG. 4B. The interface may display a plurality of route labels, and the plurality of route labels are the label of the at least one to-be-selected route. The label of the at least one to-be-selected route may be arranged in a specific order. The label of the first route may be the label 404 of the route A. If the user taps the label of the first route, the first route may be determined as the first target route.

The order of the label of the at least one to-be-selected route may be determined based on one or more of the following: slope information of a route, material information of a road surface, personal information of the user, and location information of the user. The personal information of the user may be understood as an exercise habit of the user, a personal capability of the user, and the like.

It should be noted that, in different scenarios, the order of the label of the at least one to-be-selected route may be different.

For example, when the user is currently within a range in which the user frequently exercises and familiar with surrounding routes, the label of the at least one to-be-selected route may be arranged based on an exercise habit of the user, so that a route frequently selected by the user is arranged at the very front. When the user is currently in an unfamiliar environment and unfamiliar with surrounding routes, the label of the at least one to-be-selected route may be sorted based on a distance from the user, so that a route closest to the user is arranged at the very front. When the plurality of route labels are arranged based on a road surface material, road surface material information of each route may be displayed near the route label. For example, a route A is a plastic road surface, and a route B is a cement road surface, so that the user may select a target route based on the road surface material. When the plurality of route labels are arranged based on surrounding environments of the routes, surrounding environment information of each route may be displayed near the route label. For example, a route A is a route inside a city park, and a route B offers a lake view around.

In another implementation, the first target route is automatically determined for the user.

For example, the first target route is determined based on first information, where the first target route belongs to one of the at least one to-be-selected route, and the first information includes at least one of the following: an exercise habit of the user and a personal capability of the user.

In addition, before the first target route is determined based on the first information, the at least one to-be-selected route may be further determined based on second information, where the second information includes one or more of the following: slope information of a route, material information of a road surface, a surrounding environment of a route, location information of the user, and route information of the user.

In other words, the to-be-selected routes may be a plurality of routes near a current location of the user, and the to-be-selected routes may be routes preset by the system. Manners of determining the at least one to-be-selected route based on the second information include but are not limited to the following several manners:
Manner 1: A route within a specific range away from the user is determined as the to-be-selected route based on location information of the user (for example, a current location of the user).
Manner 2: A route within a specific range from a running route of the user is determined as the to-be-selected route based on route information of the user (for example, a running route of the user within a time threshold).
Manner 3: A route within a specific range from a running route of the user is determined as the to-be-selected route based on route information of the user (for example, a running route of the user when a distance threshold is reached).

S1502: Display a first interface.

It should be noted that the first interface includes information about a first user and information about the first target route, the first user is a user of the first electronic device, the first interface further includes information about a second user, the second user and the first user are in a first exercise state, and the first exercise state is a state of exercising along the first target route. That the second user and the first user are in the first exercise state may be understood as that the second user and the first user are exercising in a group running mode for the first target route.

For example, the first interface may be the group running mode interface shown in FIG. 4E. The first user is a local user, that is, a user of the first electronic device. The first interface displays the information about the local user and the information about the first target route (for example, the route A). The first interface displays information about users who have joined the first target route, for example, the information about the first user and the information about the second user. Both the second user and the first user are users who are currently exercising in the group running mode for the first target route. For example, the user B shown in FIG. 4E is a local user, and the user A and the user C are second users.

That is, on a group running mode interface for a route, information about a user who is exercising in a group running mode for the route may be displayed. The information about the user includes information such as a real-time location of the user, current kilometers run by the user, elapsed time, a pace, energy consumed, and a heart rate.

It should be noted that, in a group running mode for a route, a user who has joined the group running mode may implement interaction on a user interface.

In an implementation, the first interface further includes an icon of the second user, where the icon of the second user is an identifier of the second user; and an operation performed by the user on the icon of the second user is received, and the second user is set as a catch-up target.

Optionally, the operation performed by the user on the icon of the second user is received, and a fourth prompt is output, where the fourth prompt is used to prompt whether to set the second user as a catch-up target.

Further, an operation performed by the user to set the second user as a catch-up target is received, and the user may be prompted with a time required to catch up with the second user.

Optionally, the icon of the second user may be the icon 409 of the user C shown in FIG. 4E. The fourth prompt may be the prompt information 410 shown in FIG. 4G.

In an implementation, the first interface further includes an icon of the second user, where the icon of the second user is an identifier of the second user; and an operation performed by the user on the icon of the second user is received, and the second user is set as a pacer.

Optionally, the operation performed by the user on the icon of the second user is received, and a fifth prompt is output, where the fifth prompt is used to prompt whether to set the second user as a pacer.

Further, an operation performed by the user to set the second user as a pacer is received, and comparison information between the first user and the second user may be prompted.

Optionally, the icon of the second user may be the icon 409 of the user C shown in FIG. 4E. The fifth prompt may be the prompt information 410 shown in FIG. 4I. The comparison information between the first user and the second user may be the prompt information 415 shown in FIG. 4J.

Certainly, the first user may not only set another user (for example, the second user) as a pacer, but also be set by another user as a pacer.

In an implementation, third information from a second electronic device is received, and a sixth prompt is output, where the sixth prompt is used to prompt the second user to set the first user as a pacer, and the second user is a user of the second electronic device.

Optionally, the sixth prompt may be the prompt information 416 shown in FIG. 4K. The third information may be request information, and is used to request to set the first user as a pacer.

In an implementation, the second user and the first user are in a race mode, and the first interface further includes one or more of the following: comparison information between the first user and the second user; ranking information of the first user; and ranking information of the second user.

Optionally, the comparison information may be the prompt information 1308 shown in FIG. 13B, and may include comparison of exercise parameters. For example, the comparison information may include kilometers run, running duration, and a pace. The ranking information may be the prompt information 1304 shown in FIG. 13B.

It should be noted that when the user joins a group running mode for a route and may possibly deviate from the route during running, the electronic device may remind the user whether guidance is needed to return to the original route, or directly guide the user to return to the original route, or remind the user whether to switch to a new route, or directly enter a free mode, or the like. It should be understood that the plurality of feedback manners of the electronic device in response to a deviation of the user from the route may be implemented independently or simultaneously.

In an implementation, if a distance between the first user and the first target route is greater than or equal to a distance threshold, a first prompt is output, where the first prompt indicates the user to return to the first target route.

Optionally, the first prompt may be the prompt information 1004 shown in FIG. 10B. When the first user deviates from the first target route by a distance greater than or equal to the distance threshold, the first electronic device may directly display navigation information, to prompt the user to return to the original route. Certainly, the first prompt may alternatively be an alert tone played by the first electronic device. A specific prompt manner is not limited in embodiments of this application.

Specifically, the first prompt includes a return route, and the return route indicates the user to return to the first target route along the return route. The return route may be determined based on location information of the first user, and the return route includes navigation information such as a route direction and a distance. For a specific manner, refer to the foregoing description of FIG. 9. Details are not described herein again.

In addition, before outputting the first prompt, the first electronic device may further output a second prompt, where the second prompt is used to prompt the user whether to return to the first target route.

Optionally, the second prompt may be the prompt information 1001 shown in FIG. 10A.

In an implementation, if a distance between the first user and the first target is greater than or equal to a distance threshold, a third interface is displayed, where the third interface includes the information about the first user and information about a second target route.

Specifically, before the third interface is displayed, the first electronic device may output a third prompt, where the third prompt is used to prompt the user whether to select the second target route, and the second target route is different from the first target route.

Optionally, the third prompt may be the prompt information 1005 shown in FIG. 10C. When the first user deviates from the first target route by a distance greater than or equal to the distance threshold, the first user may switch to a new target route and join a group running mode for the new route. The third interface may be a group running mode interface of the second target route. Similar to the first interface (the group running mode interface of the first target route), the third interface displays the information about the second target route, and further displays information about users who have joined a group running mode for the second target route.

In another implementation, if a distance between the first user and the first target route reaches a first threshold, the user is indicated to return to the first target route; and in response to a fact that the distance between the first user and the first target route is greater than or equal to a second threshold, the first user is switched to the group running mode for the second target route, where the second threshold is greater than the first threshold.

That is, as the distance by which the user deviates from the original target route increases, the electronic device may provide different feedbacks.

It should be noted that, in the group running mode, a user with a weak GPS signal cannot obtain accurate location information. In this case, location information of other surrounding users with a strong GPS signal may be used to assist the user with a weak signal in positioning.

In an implementation, when a positioning signal of the first user is less than a signal threshold, location information of the first user is determined based on location information of a plurality of third users, where the plurality of third users are in the first exercise state, exercise information of the plurality of third users remains unchanged within a time threshold, and the time threshold is a period of time before the positioning signal of the first user is less than the signal threshold.

For example, as shown in FIG. 12, the plurality of third users may be the user B and the user C, and the first user may be the user A. In a period of time before a GPS signal of the user A is weak, exercise information of users with a strong GPS signal around the user A is recorded, for example, a step frequency and an IMU relative velocity. From the plurality of users around the user A, users (the plurality of third users) whose step frequency and IMU relative velocity remain unchanged are selected as reference users, and the location information of the user A is calculated based on location information of the reference users.

Based on the foregoing solution, an embodiment of this application provides an exercise assistance method, to provide a group running mode for a user. A route for the group running mode that the user joins is determined manually or automatically. When the user deviates from the running route, an appropriate return route is matched, and the user is guided to return to the original correct target route. When a GPS signal is weak, location information of other users may be used to assist a local user in positioning. That is, in the user group running mode, services in a plurality of scenarios are provided for the user, interaction of the user during exercise is increased, and fun of assisted exercise is enhanced, thereby improving motivation of the user for exercise.

The foregoing describes in detail the exercise assistance method provided in embodiments of this application with reference to FIG. 3 to FIG. 15. In embodiments of this application, unless otherwise stated or there is a logic conflict, terms and/or descriptions in embodiments are consistent and may be mutually referenced, and technical features in different embodiments may be combined, based on an internal logical relationship thereof, to form a new embodiment.

The following describes in detail apparatuses provided in embodiments of this application with reference to FIG. 16 and FIG. 17. It should be understood that descriptions of apparatus embodiments correspond to the descriptions of the method embodiments. Therefore, for content that is not described in detail, refer to the foregoing method embodiments. For brevity, details are not described herein again.

FIG. 16 is a block diagram of an exercise assistance apparatus 1600 according to an embodiment of this application. The exercise assistance apparatus 1600 may include units configured to perform the exercise assistance method in FIG. 15.

Specifically, the exercise assistance apparatus 1600 includes a processing unit 1610 and a transceiver unit 1620.

Optionally, the exercise assistance apparatus 1600 further includes a storage unit. The storage unit may be configured to store instructions and/or data. The processing unit 1610 may read the instructions and/or the data in the storage unit, so that the exercise assistance apparatus implements related actions performed by the electronic device in the foregoing method embodiments.

The exercise assistance apparatus 1600 may be configured to perform an action performed by the electronic device in the foregoing method embodiments. In this case, the exercise assistance apparatus 1600 may be a component of the electronic device. For example, the exercise assistance apparatus 1600 may also be a chip or an integrated circuit in the electronic device. The processing unit 1610 is configured to perform a processing-related operation of the electronic device in the foregoing method embodiments, and the transceiver unit 1620 is configured to perform a receiving/sending-related operation of the electronic device in the foregoing method embodiments.

It should be understood that a specific process in which the units perform the foregoing corresponding steps is described in detail in the foregoing method embodiments. For brevity, details are not described herein again.

During specific implementation, actions performed by the processing unit 1610 and the transceiver unit 1620 may be implemented by one processor, or may be implemented by a plurality of processors.

FIG. 17 is another block diagram of an exercise assistance apparatus according to an embodiment of this application. The exercise assistance apparatus 1700 shown in FIG. 17 may include a processor 1710, a transceiver 1720, and a memory 1730. The processor 1710, the transceiver 1720, and the memory 1730 are connected through an internal connection path. The memory 1730 is configured to store instructions. The processor 1710 is configured to execute the instructions stored in the memory 1730, to implement the method in the foregoing embodiments. Optionally, the memory 1730 may be coupled to the processor 1710 through an interface, or may be integrated with the processor 1710.

It should be noted that the transceiver 1720 may include but is not limited to a transceiver apparatus like an input/output interface (input/output interface), to implement communication between the exercise assistance apparatus 1700 and another device or a communication network.

The memory 1730 may be a volatile memory and/or a nonvolatile memory. The nonvolatile memory may be a read-only memory (read-only memory, ROM), a programmable read-only memory (programmable ROM, PROM), an erasable programmable read-only memory (erasable PROM, EPROM), an electrically erasable programmable read-only memory (electrically EPROM, EEPROM), or a flash memory. The volatile memory may be a random access memory (random access memory, RAM). For example, the RAM may be used as an external cache. By way of example, but not limitation, the RAM includes a plurality of forms, such as a static random access memory (static RAM, SRAM), a dynamic random access memory (dynamic RAM, DRAM), a synchronous dynamic random access memory (synchronous DRAM, SDRAM), a double data rate synchronous dynamic random access memory (double data rate SDRAM, DDR SDRAM), an enhanced synchronous dynamic random access memory (enhanced SDRAM, ESDRAM), a synchlink dynamic random access memory (synchlink DRAM, SLDRAM), and a direct rambus random access memory (direct rambus RAM, DR RAM).

It should be noted that when the processor is a general-purpose processor, a DSP, an ASIC, an FPGA or another programmable logic device, a discrete gate or a transistor logic device, or a discrete hardware component, a memory (storage module) may be integrated into the processor.

It should further be noted that the memory described in this specification is intended to include, but is not limited to, these and any other appropriate type of memory.

The transceiver 1720 uses, for example, but is not limited to, a transceiver apparatus like a transceiver, to implement communication between the exercise assistance apparatus 1700 and another device or a communication network, to receive/send data/information used to implement the method in the foregoing embodiments.

The exercise assistance apparatus 1700 may be a chip or a circuit disposed in the foregoing electronic device.

An embodiment of this application provides a computer program product. When the computer program product is run on a device, the device is enabled to perform the technical solutions in the foregoing embodiments. An implementation principle and technical effects thereof are similar to those in the foregoing method-related embodiments. Details are not described herein again.

An embodiment of this application provides a readable storage medium. The readable storage medium includes instructions. When the instructions are run on a device, the device is enabled to perform the technical solutions in the foregoing embodiments. An implementation principle and technical effects thereof are similar. Details are not described herein again.

An embodiment of this application provides a chip. The chip is configured to execute instructions. When the chip runs, the technical solutions in the foregoing embodiments are performed. An implementation principle and technical effects thereof are similar. Details are not described herein again.

A person of ordinary skill in the art may be aware that units and algorithm steps in the examples described with reference to embodiments disclosed in this specification can be implemented by electronic hardware or a combination of computer software and electronic hardware. Whether the functions are performed by hardware or software depends on particular applications and design constraint conditions of the technical solutions. A person skilled in the art may use different methods to implement the described functions for each particular application, but it should not be considered that the implementation goes beyond the scope of embodiments of this application.

It may be clearly understood by a person skilled in the art that, for the purpose of convenient and brief description, for a detailed working process of the foregoing apparatus and units (modules), refer to a corresponding process in the foregoing method embodiments. Details are not described herein again.

In the several embodiments provided in this application, it should be understood that the disclosed device, apparatus, and method may be implemented in other manners. For example, the described apparatus embodiment is merely an example. For example, division into the units is merely logical function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in an electrical form, a mechanical form, or another form.

The foregoing units described as separate parts may or may not be physically separate, and parts displayed as units may or may not be physical units, may be located in one position, or may be distributed on a plurality of network units. Some or all of the units may be selected based on actual requirements to achieve the objectives of the solutions of embodiments.

In addition, functional units in embodiments of this application may be integrated into one processing unit, each of the units may exist alone physically, or two or more units may be integrated into one unit.

When the functions are implemented in a form of a software functional unit and sold or used as an independent product, the functions may be stored in a computer-readable storage medium. Based on such an understanding, the technical solutions in embodiments of this application essentially, or the part contributing to the conventional technology, or a part of the technical solutions may be implemented in a form of a software product. The computer software product is stored in a storage medium, and includes several instructions for instructing a computer device (which may be a personal computer, a server, a network device, or the like) to perform all or some of the steps of the methods described in embodiments of this application. The foregoing storage medium includes any medium that can store program code, such as a USB flash drive, a removable hard disk, a read-only memory (read-only memory, ROM), a random access memory (random access memory, RAM), a magnetic disk, or an optical disc.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. An exercise assistance method, wherein the method is applied to a first electronic device and comprises:
displaying a first interface, wherein the first interface comprises information about a first user and information about a first target route, the first user is a user of the first electronic device, the first interface further comprises information about a second user, the second user and the first user are in a first exercise state, and the first exercise state is a state of exercising along the first target route.

2. The method according to claim 1, wherein before displaying the first interface, the method further comprises:
determining the first target route.

3. The method according to claim 2, wherein determining the first target route comprises:
displaying a second interface, wherein the second interface displays a label of at least one to-be-selected route, and the label of the at least one to-be-selected route comprises a label of a first route; and
receiving an operation performed by the user on the label of the first route, and determining the first route as the first target route.

4. The method according to claim 3, wherein an order of the label of the at least one to-be-selected route is determined based on one or more of the following: slope information of a route, material information of a road surface, personal information of the user, and location information of the user.

5. The method according to claim 2, wherein determining the first target route comprises:
determining the first target route based on first information, wherein the first target route belongs to one of at least one to-be-selected route, and the first information comprises at least one of the following:
an exercise habit of the user and a personal capability of the user.

6. The method according to claim 5, wherein before determining the first target route based on the first information, the method further comprises:
determining the at least one to-be-selected route based on second information, wherein the second information comprises one or more of the following:
slope information of a route, material information of a road surface, a surrounding environment of a route, location information of the user, and route information of the user.

7. The method according to any one of claims 1 to 6, wherein the method further comprises:
if a distance between the first user and the first target route is greater than or equal to a distance threshold, outputting a first prompt, wherein the first prompt indicates the user to return to the first target route.

8. The method according to claim 7, wherein the first prompt comprises a return route, and the return route indicates the user to return to the first target route.

9. The method according to claim 7 or 8, wherein before outputting the first prompt, the method further comprises:
outputting a second prompt, wherein the second prompt is used to prompt the user whether to return to the first target route.

10. The method according to any one of claims 1 to 6, wherein the method further comprises:
if a distance between the first user and the first target route is greater than or equal to a distance threshold, displaying a third interface, wherein the third interface comprises the information about the first user and information about a second target route, and the second target route is different from the first target route.

11. The method according to claim 10, wherein before displaying the third interface, the method further comprises:
outputting a third prompt, wherein the third prompt is used to prompt the user whether to select the second target route.

12. The method according to any one of claims 1 to 11, wherein the first interface further comprises an icon of the second user, the icon of the second user is an identifier of the second user, and the method further comprises:
receiving an operation performed by the user on the icon of the second user, and setting the second user as a catch-up target.

13. The method according to any one of claims 1 to 11, wherein the first interface further comprises an icon of the second user, the icon of the second user is an identifier of the second user, and the method further comprises:
receiving an operation performed by the user on the icon of the second user, and setting the second user as a pacer.

14. The method according to any one of claims 1 to 13, wherein the second user and the first user are in a race mode, and the first interface further comprises one or more of the following:
comparison information between the first user and the second user;
ranking information of the first user; and
ranking information of the second user.

15. The method according to any one of claims 1 to 14, wherein the method further comprises:
when a positioning signal of the first user is less than a signal threshold, determining location information of the first user based on location information of a plurality of third users, wherein
the plurality of third users are in the first exercise state, exercise information of the plurality of third users remains unchanged within a time threshold, and the time threshold is a period of time before the positioning signal of the first user is less than the signal threshold.

16. An electronic device, wherein the electronic device is configured to perform the method according to any one of claims 1 to 15.

17. An exercise assistance apparatus, comprising a processor coupled to a memory, wherein the memory is configured to store a computer program, and the processor is configured to run the computer program, so that the exercise assistance apparatus performs the method according to any one of claims 1 to 15.

18. The exercise assistance apparatus according to claim 17, wherein the apparatus further comprises a transceiver, and the transceiver is configured to receive a signal and/or transmit a signal.

19. A computer-readable storage medium, wherein the computer-readable storage medium stores a computer program, and when the computer program is executed by a computer, the computer is enabled to perform the method according to any one of claims 1 to 15.

20. A chip, wherein the chip comprises a processor and a data interface, and the processor reads, through the data interface, instructions stored in a memory, to perform the method according to any one of claims 1 to 15.
